# EUROPÄISCHE PATENTSCHRIFT

(11) **EP 2 427 443 B1**
(45) Veröffentlichungstag und Bekanntmachung des Hinweises auf die Patenterteilung: **15.10.2014**
(21) Anmeldenummer: 10713583.2
(22) Anmeldetag: 12.04.2010
(51) Int. Cl.: C07D 307/62, A61Q 17/00, A61Q 19/00

(54) **ZIMTSÄUREASCORBATE**
CINNAMIC ACID ASCORBATES
ASCORBATE D'ACIDE CINNAMIQUE

(30) Priorität: 08.05.2009 EP 09006272
(43) Veröffentlichungstag der Anmeldung: 14.03.2012
(73) Patentinhaber: Merck Patent GmbH, 64293 Darmstadt (DE)
(72) Erfinder: RUDOLPH, Thomas, 64291 Darmstadt (DE); BUEHLE, Philipp, 64291 Darmstadt (DE)
(86) Internationale Anmeldenummer: PCT/EP2010/002242
(87) Internationale Veröffentlichungsnummer: WO 2010/127756

(56) Entgegenhaltungen:
- EP-A1- 0 664 290
- EP-A1- 1 939 192
- WO-A1-2009/097953
- WO-A2-2010/060513
- DE-A1-102006 037 724

## Beschreibung

Die Erfindung betrifft spezielle Zimtsäureascorbate und deren nicht-therapeutische Verwendung als hautbindende UV-Filter sowie ein Verfahren zu ihrer Herstellung und Zubereitungen enthaltend diese Verbindungen.

Die menschliche Haut unterliegt gewissen Alterungsprozessen, die teilweise auf intrinsische Prozesse (chronoaging) und teilweise auf exogene Faktoren (environmental, z.B. photoaging) zurückzuführen sind.

Zu den exogenen Faktoren zählen insbesondere das Sonnenlicht oder künstliche Strahlungsquellen mit vergleichbarem Spektrum sowie Verbindungen, die durch die Strahlung entstehen können, wie undefinierte reaktive Photoprodukte, die auch radikalisch oder ionisch sein können.

Es ist eine Vielzahl von organischen und anorganischen UV-Filtern und Antioxidantien bekannt, die UV-Strahlung absorbieren und freie Radikale abfangen können. Sie sind so in der Lage, die menschliche Haut zu schützen. Durch diese Verbindungen wird die Transformation von UV-Licht in Wärme katalysiert.

Aufgrund mangelnder Hauthaftung ist aber die Schutzdauer begrenzt, insbesondere weil konventionelle UV-Filter sehr leicht abgewaschen werden können, beispielsweise von Schweiß oder Wasser.
Es ist eine beispielsweise aus WO 2006/018104 bekannte Strategie, UV-Filter oder Selbstbräunersubstanzen derart zu derivatisieren, dass sie über einen reaktiven Molekülpart kovalent an das Stratum Corneum der Epidermis binden können und so die Haut mit dem UV-Filter oder Selbstbräuner funktionalisieren. Für die effektive Anbindung an Proteine und Aminosäuren der äußeren Hautschichten ist es erforderlich, dass die entsprechenden UV-Filterderivate, bzw. Derivate anderer Wirkstoffe, wie pharmakologische, antimikrobielle, fungizide, herbizide, insektizide oder kosmetische Wirkstoffe, Röntgenkontrastmittel oder Farbstoffe, über eine möglichst hohe Reaktivität ihrer bindungsfähigen Molekülteile verfügen.

Aus WO 2008/017346 sind beispielsweise Ascorbinsäurederivate bekannt, die als hautbindende UV-Filter verwendet werden können.

Es besteht jedoch weiterhin ein Bedarf nach hautverträglichen Verbindungen zum UV-Schutz, insbesondere auf Basis von Zimtsäure, die in der Lage sind, proteinhaltige Matrices zu funktionalisieren oder mit anderen Worten die in der Lage sind, mit Substanzen, die freie NH-, NH₂-, SH- oder OH-Gruppen besitzen zu assoziieren und gegebenenfalls kovalente Bindungen auszubilden, sich in geeigneter Weise in kosmetische oder pharmakologische Zubereitungen einarbeiten lassen und insbesondere sowohl hinsichtlich reversibler Photoreaktionen als auch hinsichtlich irreversibler Photoreaktion stabiler sind als bisher bekannte UV-Filter, deren Struktur auf Zimtsäure basiert.

Die Aufgabe der Erfindung war dementsprechend alternative hautverträgliche Verbindungen aufzufinden, die diesen Bedarf decken können.

Es wurde nun überraschend festgestellt, dass spezielle Zimtsäureascorbate der Formel I diese Aufgabe in besonderem Maße lösen. Es kann erfindungsgemäß sowohl D- als auch L-Ascorbinsäure oder Mischungen derselben zu Verbindungen der Formel I derivatisiert werden.

Ein erster Gegenstand der Erfindung sind daher Verbindungen der Formel I, 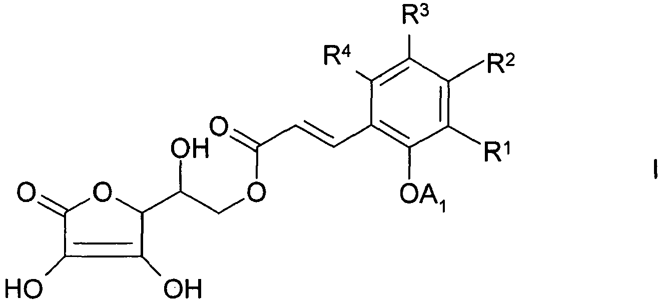 wobei
A₁ für H oder eine geradkettige oder verzweigte Alkylgruppe mit 1 bis 20 C-Atomen steht,
R¹ bis R⁴ stehen jeweils unabhängig voneinander für H, geradkettige oder verzweigte Alkoxygruppen mit 1 bis 20 C-Atomen, Hydroxy, fluorierte geradkettige oder verzweigte Alkoxygruppen mit 1 bis 20 C-Atomen oder Alkylcarbonyloxy und
Alkylcarbonyloxy steht für Alkyl-C(=O)-O, wobei Alkyl eine geradkettige oder verzweigte Alkylgruppe mit 1 bis 10 C-Atomen bedeutet.

Erfindungsgemäß können auch Derivate der Verbindungen der Formel I eingesetzt werden, die in 2- und/oder 3-Position am Ascorbinsäureteil substituiert vorliegen können. Durch geeignete Maßnahmen können diese geschützten Hydroxygruppen der Verbindungen der Formel I nach Aufbringen auf die Haut entschützt werden und so einer Hautbindung zur Verfügung stehen. Beispiele von modifizierten Hydroxygruppen in der 2-und/oder 3-Position am Ascorbinsäureteil der Verbindungen der Formel I sind -O-Alkyl, -OC(O)-Alkyl, -OPO₃M oder O-Glycosyl, wobei Alkyl eine geradkettige oder verzweigte Alkylgruppe mit 1 bis 20 C-Atomen bedeutet und M Alkalimetallkation, Erdalkalimetallkation oder H bedeutet.

Die Anbindung eines Kohlenhydrates in Position 2 oder 3 der Ascorbinsäure, zuvor als O-Glycosyl bezeichnet, kann beispielsweise für Monosaccharide wie Ribose, Arabinose Xylose, Lyxose, Allose, Altrose, Glucose, Mannose, Gulose, Idose, Galaktose, Talose, Ribulose, Xylulose, Psicose, Fructose, Sorbose oder Tagatose erfolgen. In dieser Aufzählung sind beide Isomeren, d.h. jeweils die D- oder L-Formen enthalten. Vorzugsweise werden Glucose, Galactose oder Fructose, ganz besonders bevorzugt Glucose, verwendet.

Prinzipiell sind jedoch auch Disaccharide geeignet, wie Saccharose (oder auch Sucrose genannt), Lactose, Trehalose, Maltose, Cellobiose, Gentiobiose oder Melibiose. In dieser Aufzählung sind sowohl die α- als auch die β-Formen enthalten.
Aus der Gruppe der Disaccharide werden vorzugsweise Saccharose oder Lactose, insbesondere bevorzugt Saccharose verwendet.

Aus EP 664290 sind ähnliche Zimtsäureascorbate bekannt, beispielsweise Di-Zimtsäure-2O,6O-Ascorbat. Die Synthese dieser Verbindungen kann unter anderem über Mono-Zimtsäure-6-O-ascorbate erfolgen, die durch die allgemeine Strukturformel 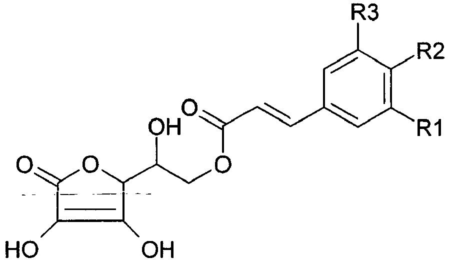 beschrieben werden, wobei R1 bis R3 unabhängig voneinander H, Alkoxy, Hydroxy, Alkylcarbonyloxy oder Fluoralkoxy bedeuten können. Es wird in EP 664290 jedoch kein Beispiel eines solchen Mono-Zimtsäure-6-O-ascorbats offenbart.

JP 2009-035509 beschreibt die enzymatische Synthese von Mono-Zimtsäure-6-O-ascorbaten der Struktur 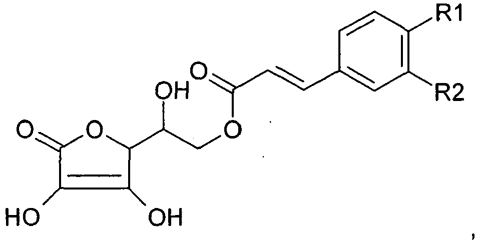 wobei R1 und R2 unabhängig voneinander H, Hydroxy oder niedriges Alkoxy bedeuten können.

WO 2008/017346 beschreibt ebenfallss ähnliche Mono-Zimtsäure-6O-ascorbate einer allgmeinen breiten Formel, wobei jedoch als Einzelverbindung 4-Methoxyzimtsäure-6-O-ascorbat offenbart ist.

Die erfindungsgemäßen Verbindungen der Formel I unterscheiden sich prinzipiell von allen bisher bekannten Mono-Zimtsäure-6-O-ascorbaten durch den orthoständigen Substituenten OA₁, der insbesondere für die Vorteile der erfindungsgemäßen Verbindungen verantwortlich ist.

Die erfindungsgemäßen Substanzen der Formel I zeigen insbesondere hinsichtlich reversibler Photoreaktionen gegenüber den bekannten Zimtsäurederivaten Vorteile auf.

Für bekannte Zimtsäurederivate ist beispielsweise die trans,cis-Photoisomerisierung als reversible Photoreaktion bekannt. Durch UV-Licht kann das thermodynamisch stabilere trans-Isomere mit dem cis-Isomeren (= Photoisomeres) in das sog. photostationäre Gleichgewicht gesetzt werden.

So weiß der Lichtschutzexperte z.B., dass der weltweit verwendete kosmetische UV-Filter Ethylhexylmethoxycinnamat EHMC (e.g. Eusolex® 2292) in seiner Syntheseform als reines trans-Isomeres vorliegt. Durch UV-Bestrahlung in Lösung oder im Kosmetikum stellt sich je nach Matrixgegebenheiten und Spektralcharakteristik der UV-Quelle ein photostationäres Gleichgewicht ein, in dem der Anteil des trans-isomeren in der Regel bei nurmehr 40-60% liegt. Parallel entstehen 60-40% cis-Isomeres (= Photoisomeres). Dieser Sachverhalt ist auch für die Mono-Zimtsäure-6O-ascorbate aus dem Stand der Technik zu erwarten bzw am Beispiel des 3,4-Dimethoxyzimtsäure-6-O-ascorbats (3-(3,4-Dimethoxy-phenyl)-acrylsäure-(S)-2-((R)-3,4-dihydroxy-5-oxo-2,5-dihydrofuran-2-yl)-2-hydroxy-ethylester) stellvertretend belegt und in den Beispielen 5 und 6 beschrieben.

Charakteristisch für cis-Photoisomere ist, dass ihre Absorptionskraft im Vergleich zum trans-Isomeren reduziert ist. Für cis-EHMC ist die spezifische Extinktion am Extinktionsmaximum stark reduziert (ε = 13500, λmax(MeOH)[nm] = 304). Demgegenüber ist die spezifische Extinktion von trans-EHMC deutlich höher (ε = 24550, λmax(MeOH)[nm] = 309). [Siehe hierzu: M. Köhnlein: "Untersuchungen zum photochemischen Verhalten des UVB-Filters Octyl-methoxycinnamat in Modellsystemen, Sonnenschutzmitteln sowie auf der Haut"; Thesis Universität Hohenheim 2000; ISBN 3-8265-8234-9].

Für den Kosmetikchemiker ist es daher erstrebenswert, dass möglichst wenig Photoisomeres entsteht, da dessen spezifische Absorptionsfähigkeit im Vergleich zum trans-Isomeren stark reduziert ist und somit unter Bestrahlungsbedingungen die integrale Absorptionskraft beider Isomere geschwächt wird.

Die erfindungsgemäße Substanzen der Formel I weisen hinsichtlich der reversiblen Photoreaktion ein stark verbessertes Photoverhalten auf, woraus sich für die praktische Anwendung im UV-Schutz erhebliche Vorteile der erfindungsgemäßen Verbindungen hinsichtlich der Stabilität in Zubereitungen ergeben.

In den Verbindungen der Formel I bedeutet die geradkettige oder verzweigte Alkylgruppe mit 1 bis 20 C-Atomen beispielsweise Methyl, Ethyl, Propyl, n-Butyl, tert.-Butyl, n-Pentyl, n-Hexyl, 2-Ethyl-hexyl, n-Dodecyl oder n-Lauryl. Bevorzugt verwendet wird eine geradkettige oder verzweigte Alkylgruppe mit 1 bis 4 C-Atomen.

In den Verbindungen der Formel I bedeutet die geradkettige oder verzweigte Alkoxygruppe mit 1 bis 20 C-Atome beispielsweise Methoxy, Ethoxy, Propoxy, Isopropoxy, Butoxy, Isobutoxy, Tert.-Butoxy, n-Pentoxy, n-Hexyloxy, n-Heptyloxy, n-Octyloxy, n-Ethylhexyloxy, n-Dodecyloxy oder n-Lauryl-oxy. Vorzugsweise hat die Alkoxygruppe 1 bis 6 C-Atome, besonders bevorzugt 1 bis 4 C-Atome. Ganz besonders bevorzugt ist die Alkoxygruppe Methoxy.

Fluorierte geradkettige oder verzweigte Alkoxygruppen entspechen den soeben beschriebenen Alkoxygruppen, wobei die H-Atome teilweise oder vollständig durch F ersetzt werden, beispielsweise Trifluormethoxy, Pentafluorethoxy, Trifluormethylethoxy, Heptafluorpropoxy oder Nonafluorbutoxy.

A₁ in Formel I bedeutet vorzugsweise eine geradkettige oder verzweigte Alkylgruppe mit 1 bis 20 C-Atomen, besonders bevorzugt eine geradkettige oder verzweigte Alkylgruppe mit 1 bis 4 C-Atomen, ganz besonders bevorzugt ist ist A₁ Methyl.

R² in Formel I bedeutet vorzugsweise H, Hydroxy oder eine geradkettige oder verzweigte Alkoxygruppe mit 1 bis 20 C-Atomen. Besonders bevorzugt ist R² Hydroxy oder eine geradkettige oder verzweigte Alkoxygruppe mit 1 bis 4 C-Atomen. Ganz besonders bevorzugt ist R² eine Alkoxygruppe mit 1 bis 4 C-Atomen.

R¹, R³ und R⁴ sind jeweils unabhängig voneinander vorzugsweise H, Hydroxy oder eine geradkettige oder verzweigte Alkoxygruppe mit 1 bis 20 C-Atomen.

In einer bevorzugten Ausführungsform der Verbindungen der Formel I bedeutet mindestens ein weiterer Subsitutent ausgewählt aus R¹, R³ oder R⁴ Hydroxy oder eine geradkettige oder verzweigte Alkylgruppe mit 1 bis 20 C-Atomen, vorzugsweise eine geradkettige oder verzweigte Alkylgruppe mit 1 bis 4 C-Atomen.

In Summe ist es bevorzugt, wenn mindestens zwei geradkettige oder verzweigte Alkoxygruppen mit C-Atomen, wie zuvor beschrieben am Benzolring vorhanden sind. Besonders bevorzugt sind drei geradkettige oder verzweigte Alkoxygruppen mit C-Atomen, wie zuvor beschrieben am Benzolring vorhanden.

Besonders bevorzugte Verbindungen der Formel I sind
2,4-Dimethoxyzimtsäure-6-O-ascorbat,
2,4,6-Trimethoxyzimtsäure-6-O-ascorbat,
2,3,4-Trimethoxyzimtsäure-6-O-ascorbat,
2,4,5-Trimethoxyzimtsäure-6-O-ascorbat.

Im Gegensatz zu dem bekannten Zimtsäurederivat 4-Methoxyzimtsäure-6-O-ascorbat, dessen UV-Absorption im UVB-Bereich liegt (per Definition liegt das UV-Absorptionsmaximum zwischen 290 nm und 320 nm), ist es bevorzugt, wenn erfindungsgemäße UV-Filter verstärkt im UVA-Bereich absorbieren. Im Idealfall liegt dabei das UV-Absorptionsmaximum im Bereich zwischen 320 nm und 400 nm. Die erfindungsgemäßen Verbindungen der Formel I, oder die als bevorzugt beschriebenen Verbindungen, wie zuvor beschrieben, sind insbesondere hautbindende und/oder haarbindende UVA-Filter.

Ein weiterer Gegenstand der Erfindung ist daher auch die nicht-therapeutische Verwendung der erfindungsgemäßen Verbindungen der Formel I, wie zuvor beschrieben als hautbindender UV-Filter, insbesondere als hautbindender UVA-Filter.

Die Herstellung generell der Verbindungen der Formel I kann durch Veresterung erfolgen.

Ein weiterer Gegenstand der Erfindung ist daher ein Verfahren zur Herstellung der erfindungsgemäßen Verbindungen der Formel I, wie zuvor beschrieben, dadurch gekennzeichnet, dass Ascorbinsäure mit einer Verbindung der Formel II, 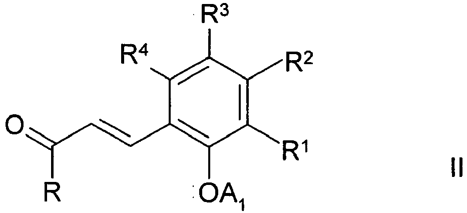 verestert wird,
wobei R OH, Halogen oder ein Aktivester bedeutet, Halogen Cl, Br oder I bedeutet und die Substituenten A₁, R¹ bis R⁴ eine zuvor definierte Bedeutung haben.

Ascorbinsäure ist kommerziell erhältlich. Die Verbindungen der Formel II sind teilweise kommerziell erhältlich oder können nach Methoden synthetisiert werden, die z.B. in den Standardwerken wie Houben-Weyl, Methoden der organischen Chemie, Georg-Thieme-Verlag, Stuttgart beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die direkte Veresterung der Verbindungen der Formel II mit Ascorbinsäure, wobei in Formel II R = OH bedeutet, erfolgt beispielsweise in Gegenwart von konzentrierter Schwefelsäure und vorzugsweise unter Inertgasbedingungen. Vorteilhaft wird die Mischung der Komponenten bei Temperaturen < 5°C hergestellt. Die eigentliche Reaktionstemperatur liegt zwischen 10 und 60°C, bevorzugt zwischen 15 und 30°C. Besonders bevorzugt erfolgt die Umsetzung bei Raumtemperatur.

Anstelle der freien Säure der Formel II, wie zuvor definiert für R = OH, können auch Derivate der Formel eine voraktivierte Säure, oder ein Säurehalogenid (R = Halogen, vorzugsweise Cl), ein symmetrisches oder gemischtes Anhydrid oder ein Aktivester, eingesetzt werden. Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart) beschrieben. Aktivierte Ester werden zweckmäßig in situ aus Verbindungen der Formel I mit R = OH gebildet, z.B. durch Zusatz von HOBt (1-Hydroxybenzotriazol) oder N-Hydroxysuccinimid.

Die Umsetzung erfolgt in der Regel in einem inerten Lösungsmittel, bei Verwendung eines Halogenids der Formel II in Gegenwart eines säurebindenden Mittels vorzugsweise einer organischen Base wie Triethylamin, Dimethylanilin, Pyridin, Dimethylaminopyridin oder Chinolin.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Auch enzymatische Veresterungen, beispielsweise durch Lipasen sind geeignet.

Bei der direkten Veresterung entsteht synthesebedingt ein Gemisch aus Ascorbinsäure-C-6-Estern und Ascorbinsäure-C-5-Estern, wobei in der Regel der Ascorbinsäure-C-6-Ester überwiegt, d.h. Verbindungen der Formel I. Auch diese Mischungen können direkt in die erfindungsgemäßen Zubereitungen eingearbeitet werden. Selbstverständlich sind diese Gemische über Methoden trennbar, die dem Fachmann bekannt sind, so dass die reinen Verbindungen der Formel I isolierbar sind. Je nach Anteil des entsprechenden Ascorbinsäure-C-5-Esters ist eine Isolierung für die erfindungsgemäße Anwendung jedoch nicht notwendig.
Die freien Hydroxygruppen der Ascorbinsäure in 2- und 3-Position können wahlweise auch vor der eigentlichen Veresterung mit Schutzgruppen blockiert werden, falls dies notwendig erscheint.

Die Ascorbinsäure-C-5-ester der Verbindungen der Formel I können durch folgende Formel I-1 dargestellt werden 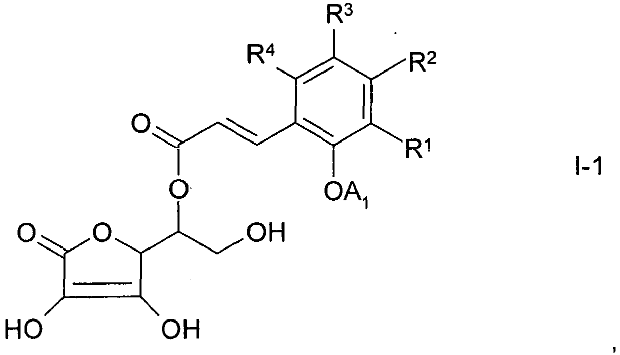 wobei die Substituenten A₁, R¹ bis R⁴ eine der zuvor genannten Bedeutungen haben. Es gelten für die Verbindungen der Formel-1 die gleichen Ausführungen zu den möglichen Substituenten und Abbauprodukten, wie für die Verbindungen der Formel I beschrieben.

Für die Bindung erfindungsgemäßer Substanzen an Proteine oder protein- oder aminosäurehaltige Matrices, wie das Stratum Corneum der Haut, ist es von Vorteil, wenn der Ascorbinsäureanteil einer der Ascorbinsäure-Maillardreaktion vergleichbaren Abbaureaktion unterliegt. Dieser Abbau ist durch typische gegebenenfalls aufeinanderfolgende Reaktionen gekennzeichnet. Solche typischen Reaktionen, denen die Ausgangssubstanz demnach unterworfen sein kann, können z. B. die Dehydrogenierung, Dehydratisierung, Hydratisierung, Hydrolyse, Oxidation, Isomerisierung und/oder Eliminierung (z.B. von Wasser und/oder Kohlendioxid) umfassen. Hierbei ist insbesondere die Oxidation zu entsprechenden Dehydroascorbaten als Initialschritt anzunehmen.

Nachfolgend sind exemplarisch Strukturen aufgelistet, die über Maillarrreaktion-analoge Mechanismen postulierbar sind (primäre Maillardprodukte). Exemplarische Strukturen sind als Intermediate aufzufassen, die an Proteine des Stratum Corneum binden können.

Die Entstehung aufgeführter exemplarischer Strukturen ist gleichzeitig als Aktivierung der Ausgangssubstanzen aufzufassen, weil hierduch die chemische Reaktivität bezüglich der Reaktion mit Proteinen gesteigert werden kann.

Demzufolge kann es das Ziel sein, je nach Applikation diese Strukturen durch chemische Reaktion gezielt aus der Ausgangssubstanz entstehen zu lassen, um eine gesteigerte Proteinbindungsreaktion zu erzielen. Bevorzugt wird dabei die Abbaureaktion durch Oxidation eingeleitet. Oxidation kann durch Exposition gegenüber Sauerstoff/Luft initiiert werden oder auch z.B. durch Photooxidation erfolgen in Gegenwart geeigneter Strahlung, wie z.B. UV-Licht oder Sonnenstrahlung. Oxidation kann aber auch durch gezielte Verwendung von Oxidationsmitteln hervorgerufen werden. Als Oxidationsmittel kommen exemplarisch z.B. Peroxide, wie Wasserstoffperoxid oder Hydroperoxide, Ozon, Radikale, Reaktive Sauerstoffspezies oder anorganische Oxidantien wie EisenIII/II- oder Kupferll/I-Salze in Frage.

Besagte Aktivierung durch Oxidantien kann z.B. dadurch erreicht werden, indem man eine das Oxidans enthaltende Zubereitung und eine zweite, die Ausgangssubstanz enthaltende Zubereitung, unmittelbar vor der Applikation auf Haut oder Haar miteinander vermischt oder z.B. die Haut zeitlich versetzt zunächst mit der zweiten, die Ausgangssubstanz enthaltenden Zubereitung behandelt, um nachfolgend die das Oxidans enthaltende erste Zubereitung aufzutragen.

Oxidationsreaktionen durch Luftsauerstoff nach erfolgter Applikation treten dann mit hohem Umsatz auf, wenn die Applikationsschicht möglichst dünn aufgetragen wird. Hohen Einfluß hat dabei aber auch die Zubereitung bzw. das Lösungsmittel, in dem erfindungsgemäße Substanzen appliziert werden. Insbesondere polarprotische Lösungsmittel, wie Wasser, Glycole und Glycolderivate (e.g. Glycerin, Ethylenglycol, Polyethylenglycole) fördern die Hautanbindung. Zudem kann es hilfreich sein, nach Applikation die Substanzumgebung zu alkalisieren, um die Hautanbindung zu beschleunigen. Denkbar ist es zudem, bereits vor Applikation der Ausgangssubstanz entweder das Auftragsareal zu alkalisieren, oder die die Ausgangssubstanz enthaltende Zubereitung durch Zugabe einer weiteren Zubereitung zu alkalisieren. Unter dem Begriff "Alkalisieren" versteht man allgemein, den in der Zubereitung vorliegenden, in der Regel möglichst niedrigen Umgebungs-pH des Ascorbinsäurederivates anwendungsspezifisch ad-hoc zu erhöhen, um dadurch dessen Oxidationsempflindlichkeit und die damit verbundene Proteinbindungsfähigkeit gezielt zu steigern (e.g. durch Behandlung mit NaOH oder anderen chemischen Basen)

Sogenannte primäre Maillardprodukte gemäß Tabelle sind ebenso wie die Ausgangssubstanzen als individuelle haut- und haarbindende UV-Filter aufzufassen und mit den Ausgangssubstanzen, d.h. beispielsweise den Verbindungen der Formel I oder deren cis-Isomeren oder deren 50-Ascorbaten kombinierbar.

### Tabelle: exemplarisch postulierbare frühe Maillardprodukte von Mono-Zimtsäureascorbaten mit Proteinbindungsfähigkeit

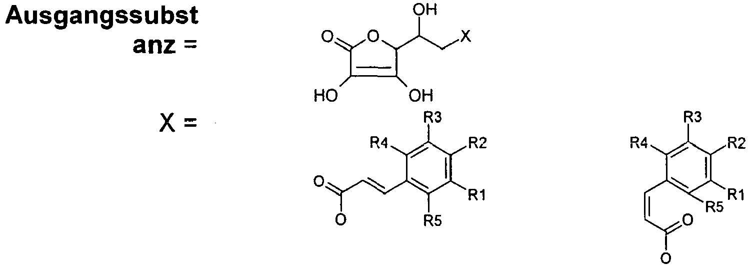

wobei R1-R5 unabhängig voneinander H, Alkoxy, Hydroxy, Alkylcarbonyloxy, oder Fluoroalkoxy repräsentieren, wie zuvor beschrieben und für die erfindungsgemäßen Ausgangssubstanzen R5 = OA₁ bedeutet, wie zuvor beschrieben. Ebenfalls gilt für die Definitionen der Substituenten R1 bis R5 die vorherige Beschreibung entsprechend. 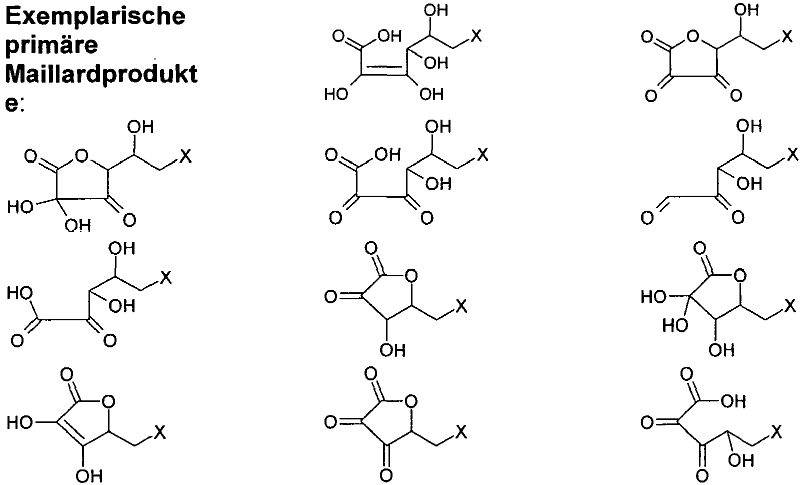 Die exemplarisch hier aufgeführten primären Abbauprodukte der Ausgangssubstanzen können weiteren chemischen Veränderungen unterliegen, bevor sie mit den Proteinen e.g. von Haut und Haar reagieren. Diese Veränderungen lassen sich exemplarisch als Dehydrogenierung, Dehydratisierung, Hydrolyse, Oxidation, Isomerisierung, Hydratisierung und/oder Eliminierung (e.g. von Wasser und/oder Kohlendioxid) beschreiben. Analoge Betrachtungen sind für die entsprechenden isomeren Ascorbinsäure-5O-Ester anzustellen.

Die erfindungsgemäßen Substanzen der Formel I, sowie z.B. auch 4-Methoxycinnamoyl-6O-ascorbat, zeichnen sich neben ihrem hohen Hautbindungsvermögen auch zusätzlich dadurch aus, dass sie in der Umwelt biologisch gut abbaubar sind.

Weitere Vorteile erfindungsgemäßer Substanzen, sowie z.B. auch von 4-Methoxycinnamoyl-6O-ascorbat, sind in ihrer antimikrobiellen Wirksamkeit zu sehen. Ferner eignen sich diese Substanzen zur Verbesserung der Hautbarriere. Sie können wundheilende Wirkung besitzen, ggbf. gegen Cellulite und die verschiedenen Formen der Akne wirken. Ihre Anti-aging-Wirkung ist nicht nur auf ihre UV-filternde Wirkung zurückzuführen, sondern geht zudem auf Wirkweisen zurück, wie sie für Ascorbinsäure vorbeschrieben wurden. Neben der Inhibierung von sog. Matrixmetalloproteinasen (zusätzlicher anti-aging Effekt) können beschriebene Substanzen auch die Hautfarbe bzw. Haarfarbe beeinflussen. Insbesondere in Kombination mit klassischen Selbstbräunungssubstanzen wie Dihydroxyaceton oder Erythrulose (aber auch in deren Abwesenheit) können gewünschte Effekte wie Bräunungsintensivierung, Bräunungsverlängerung, oder Erhöhung des Rotanteils im Bräunungsbild erzielt werden. Da die hautaufhellende Wirkung der Ascorbinsäure bekannt ist, kann auch mit den hier beschriebenen Substanzen, ggbf. in synergistischer Kombination mit bekannten Hautaufhellern, hautaufhellende Wirkung erzielt werden.

Auf Grund ihrer antioxidativen Eigenschaften eignen sich die erfindungsgemäßen Substanzen der Formel I hervorragend als Produktschutzkomponenten zur Verhinderung des oxidativen Abbaus von sensitiven Rezepturbestandteilen in Zubereitungen, wie Farbstoffen, Parfümkomponenten oder Vitaminen. Sie können mit nachfolgend exemplarisch beschriebenen Duftstoffen vergesellschaftet sein bzw. diese stabilisieren:
Alle Duftstoffe wie beschrieben in "S. Arctander, Perfume and Flavor Materials, Vol. I and II, Montclair, N.J., 1969, Selbstverlag" oder in "K. Bauer, D. Garbe and H. Surburg, Common Fragrance and Flavor Materials, 4th Ed., Wiley-VCH, Weinheim 2001". Alle Duftstoffe wie beschrieben in US7354893 B2.

Ein weiterer Gegenstand der Erfindung ist daher auch die Verwendung von Verbindungen der Formel I, wie zuvor beschrieben, zum Produktschutz von sensitiven Zubereitungsbestandteilen.

Erfindungsgemäße Substanzen können als partikuläre Darreichungsform bereitgestellt werden. Dabei können die durchschnittlichen Partikelgrößen bei 0.001-0.1 µm, bevorzugt bei 0.1-5 µm liegen. Besonders bevorzugt ist eine Verteilung mit einem d50 Wert (laser diffraction) von 100 nm-1 µm. Die Wirkstoffe können allein oder abgemischt mit Trägermaterialien wie z.B. Sorbitol oder Mannitol bereitgestellt werden. Die Substanzen können in dieser Dareichungsform bei topischer Applikation eine Depotwirkung entfalten und geben den Wirkstoff nach und nach an die Haut ab. Dies geschieht mittels der follikulären, transzellulären, sowie Interzellulären (Korneocyten) Penetrationsroute. Die erfindungsgemäßen Substanzen können ferner in Form von öligen Abmischungen mit typischen Ölen/Emollients der Pharma oder Kosmetikindustrie bereitgestellt werden. Dabei kommt es zur Verwendung von Dispergierhilfsmitteln.

Ein weiterer Gegenstand der vorliegenden Erfindung sind Zubereitungen, die mindestens eine Verbindung der Formel I mit den zuvor beschriebenen oder als bevorzugt angegebenen Verbindungen der Formel I oder die gelisteten Einzelverbindungen enthalten.

Bei den Zubereitungen handelt es sich dabei üblicherweise um topisch anwendbare Zubereitungen, beispielsweise kosmetische oder dermatologische Formulierungen oder Medizinprodukte. Die Zubereitungen enthalten in diesem Fall einen kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe.
Topisch anwendbar bedeutet im Sinne der Erfindung, dass die Zubereitung äußerlich und örtlich angewendet wird, d.h. dass die Zubereitung geeignet sein muss, um beispielsweise auf die Haut aufgetragen werden zu können. Bevorzugte Zubereitungen sind kosmetische Zubereitungen.

Im Sinne der vorliegenden Erfindung wird neben dem Begriff Zubereitung gleichbedeutend auch der Begriff Mittel oder Formulierung verwendet.

Die Zubereitungen können die genannten notwendigen oder optionalen Bestandteile umfassen oder enthalten, daraus im wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Weitere bevorzugte Kombinationen von Ausführungsformen sind in den Ansprüchen offenbart.

Gegenstand der Erfindung ist auch ein Verfahren zur Herstellung einer Zubereitung, wie zuvor beschrieben, wobei mindestens eine Verbindung der Formel I mit einem Träger und gegebenenfalls mit weiteren Aktiv- oder Hilfsstoffen vermischt wird. Geeignete Trägerstoffe sowie Aktiv- oder Hilfsstoffe sind im nachfolgenden Teil ausführlich beschrieben.

In bevorzugten Ausführungsformen wird die mindestens eine Verbindung der Formel I mit den definierten oder als bevorzugt angegebenen Substituenten oder bevorzugte Einzelverbindungen in den erfindungsgemäßen Zubereitungen typischerweise in Mengen von 0,05 bis 10 Gew.-%, bevorzugt in Mengen von 0,1 Gew.-% bis 5 Gew.-% und besonders bevorzugt in Mengen von 0,5 bis 2 Gew.-%, eingesetzt. Dabei bereitet es dem Fachmann keinerlei Schwierigkeiten die Mengen abhängig von der beabsichtigten Wirkung der Zubereitung entsprechend auszuwählen.

In den beschriebenen Zubereitungen, die erfindungsgemäß mindestens eine Verbindung der Formel I enthalten, können weiterhin auch Farbpigmente enthalten sein, wobei der Schichtaufbau der Pigmente nicht limitiert ist.

Vorzugsweise sollte das Farbpigment bei Einssatz von 0,5 bis 5 Gew.-% hautfarben oder bräunlich sein. Die Auswahl eines entsprechenden Pigments ist für den Fachmann geläufig.

Bevorzugte Zubereitungen enthalten neben den Verbindungen der Formel I sowie den gegebenenfalls anderen Inhaltsstoffen weitere organische UV-Filter, sogenannte hydrophile oder lipophile Sonnenschutzfilter, die im UVA-Bereich und/oder UVB-Bereich und(/oder IR und/oder VIS-Bereich (Absorber) wirksam sind. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben. Im Folgenden werden die genannten UV-Filter meist nach der INCI-Nomenklatur benannt.

Insbesondere für eine Kombination geeignet sind: para-Aminobenzoesäure und deren Derivate: PABA, Ethyl PABA, Ethyl dihydroxypropyl PABA, Ethylhexyl dimethyl PABA, z. B. vetrieben unter dem Namen "Escalol 507" von der Fa. ISP, Glyceryl PABA, PEG-25 PABA, z. B. vetrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vetrieben unter dem Namen "Neo Heliopan OS" von der Fa. Haarmann and Reimer, Dipropylene glycol salicylate, z. B. vetrieben unter dem Namen "Dipsal" von der Fa. Scher, TEA salicylate, z. B. vetrieben unter dem Namen "Neo Heliopan TS" von der Fa. Haarmann and Reimer.

β,β-Diphenylacrylate Derivate: Octocrylene, z. B. vetrieben unter dem Namen "Eusolex OCR" der Fa. Merck, "Uvinul N539" von der Fa. BASF, Etocrylene, z. B. vetrieben unter dem Namen "Uvinul N35" von der BASF.

Benzophenone Derivate: Benzophenone-1, z. B. vetrieben unter dem Namen "Uvinul 400"; Benzophenone-2, z. B. vetrieben unter dem Namen "Uvinul D50" ; Benzophenone-3 oder Oxybenzone, z. B. vetrieben unter dem Namen "Uvinul M40";Benzophenone-4, z. B. vetrieben unter dem Namen "Uvinul MS40"; Benzophenone-9, z. B. vetrieben unter dem Namen "Uvinul DS-49" von der Fa. BASF, Benzophenone-5, Benzophenone-6, z. B. vetrieben unter dem Namen "Helisorb 11" von der Fa. Norquay, Benzophenone-8, z. B. vetrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vetrieben unter dem Namen "Mexoryl SD" von der Fa. Chimex, 4-Methylbenzylidenecamphor, z. B. vetrieben unter dem Namen "Eusolex 6300" von der Fa. Merck, Benzylidenecamphorsulfonsäure, z. B. vetrieben unter dem Namen "Mexoryl SL" von der Fa. Chimex, Camphor benzalkonium methosulfate, z. B. vetrieben unter dem Namen "Mexoryl SO" von der Fa. Chimex, Terephthalylidenedicamphorsulfonsäure, z. B. vetrieben unter dem Namen "Mexoryl SX" von der Fa Chimex, Polyacrylamidomethylbenzylidenecamphor vetrieben unter dem Namen "Mexoryl SW" von der Fa. Chimex.

Phenylbenzimidazol Derivate: Phenylbenzimidazolesulfonsäure, z. B. vetrieben unter dem Namen "Eusolex 232" von der Fa. Merck, Dinatrium phenyl dibenzimidazole tetrasulfonat, z. B. vetrieben unter dem Namen "Neo Heliopan AP" von der Fa. Haarmann and Reimer.

Phenylbenzotriazol Derivate: Drometrizole trisiloxane, z. B. vetrieben unter dem Namen "Silatrizole" von der Fa. Rhodia Chimie, Methylenebis(benzotriazolyl)tetramethylbutylphenol in fester Form, z. B. vetrieben unter dem Namen "MIXXIM BB/100" von der Fa. Fairmount Chemical, oder in mikronisierter Form als wässrige Dispersion, z. B. vetrieben unter dem Namen "Tinosorb M" von der Fa. Ciba Specialty Chemicals.

Triazin Derivate: Ethylhexyltriazone, z. B. vetrieben unter dem Namen "Uvinul T150" von der Fa. BASF, Diethylhexylbutamidotriazone, z. B. vetrieben unter dem Namen "Uvasorb HEB" von der Fa. Sigma 3V, 2,4,6-tris(diisobutyl 4'-aminobenzalmalonate)-s-triazine oder 2,4,6-Tris-(biphenyl)-1,3,5-triazine.

Anthranilin Derivate: Menthyl anthranilate, z. B. vetrieben unter dem Namen "Neo Heliopan MA" von der Fa. Haarmann and Reimer.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vetrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vetrieben unter dem Namen Uvasorb K2A von der Fa. Sigma 3V und Mischungen dieses enthaltend.

Piperazinderivate wie beispielsweise die Verbindung

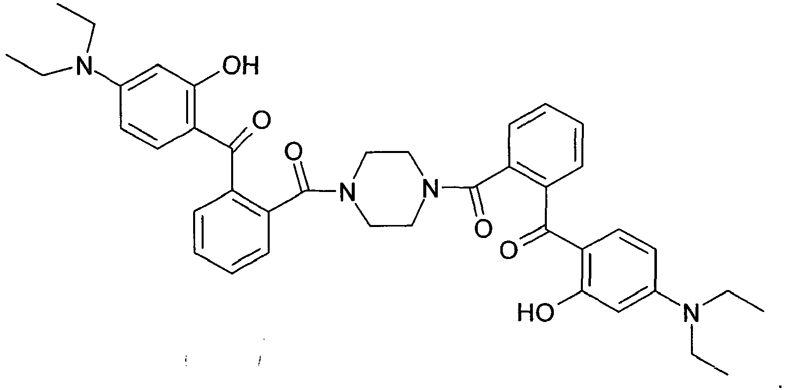

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Bevorzugte Zubereitungen enthalten neben den Verbindungen der Formel I sowie den gegebenenfalls anderen organischen UV-Filtern, wie zuvor beschrieben, weitere anorganische UV-Filter, sogenannte partikuläre UV-Filter.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex^{®} T-2000, Eusolex^{®}T-AQUA, Eusolex^{®}T-AVO, Eusolex^{®}T-OLEO), Zinkoxide (z.B. Sachtotec^{®}), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitec® COS.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53-64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt eingesetzte partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexametaphosphate, wie z.B. das Produkt "Microtitanium Dioxide MT 150 W" der Fa. Tayca.

Die zur Kombination eingesetzten behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Kemira,
- Aluminium und Glycerin; wie z.B. das Produkt UV-Titan der Fa. Kemira,
   - Aluminium und Silikonölen, wie z.B. das Produkt UV-Titan M262 der Fa. Kemira,
   - Natriumhexamethaphosphat und Polyvinylpyrrolidon,
   - Polydimethylsiloxane, wie z.B. das Produkt 70250 Cardre UF TiO2SI3" der Fa. Cardre,
   - Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   o "Zinc Oxide CS-5" der Fa. Toshibi (ZnO nachbehandelt mit polymethylhydrogenosiloxane)
   o Nanogard Zinc Oxide FN der Fa. Nanophase Technologies
   o "SPD-Z1" der Fa Shin-Etsu (ZnO nachbehandelt mit einem Silikongepfropften Acrylpolymer, dispergiert in Cyclodimethylsiloxane
   o "Escalol Z100" der Fa ISP (Aluminiumoxid nachbehandeltes ZnO dispergiert in einer ethylhexyl methoxycinnamate/PVPhexadecene/methicone copolymer Mischung)
   o "Fuji ZNO-SMS-10" der Fa. Fuji Pigment (ZnO nachbehandelt mit Siliciumdioxid und Polymethylsilesquioxan);
   o Unbehandeltes Ceroxide Mikropigment z.B. mit der Bezeichnung "Colloidal Cerium Oxide" der Fa Rhone Poulenc
   o Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandelten Titandioxid. Zinkoxid-Mischungen wie z.B . das Produkt UV-Titan M261 der Fa. Kemira in Kombination mit dem erfindungsgemäßen UV-Schutzmittel verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen. Im Einzelnen ergeben sich die folgende Vorteile:
- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es bevorzugt, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Kapselwände können auch aus PMMA bestehen. Besonders bevorzugt einzusetzende Kapseln weisen Wände auf, die durch einen SolGel-Prozess, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäß einzusetzenden Zubereitungen vorzugsweise in solchen Mengen enthalten, die gewährleisten, dass die verkapselten UV-Filter in den oben angegebenen Gewichtsprozentverhältnissen in der Zubereitung vorliegen.

Bevorzugte Zubereitungen können ebenfalls mindestens ein weiteres Ascorbinsäurederivat, bevorzugt aus der Gruppe Ascorbinsäure, Magnesiumascorbylphosphat oder Ascorbylpalmitat, enthalten.

Bevorzugte Zubereitungen können auch mindestens einen weiteren kosmetischen Wirkstoff enthalten, beispielsweise ausgewählt aus Antioxidantien, Anti-aging-Wirkstoffen, Anti-Cellulite Wirkstoffen, Selbstbräunungssubstanzen, hautaufhellenden Wirkstoffen oder Vitaminen.

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

EDTA, insbesondere Dinatrium EDTA, ist auch ein Chelatbildner und hat vorzugsweise in Kombination mit den erfindungsgemäßen Verbindungen der Formel I oder auch mit 4-Methoxycinnamoyl-6O-ascorbat die Eigenschaft, die Verbindungen der Formel I oder 4-Methoxycinnamoyl-6O-ascorbat in der Zubereitung zu stabilisieren und/oder die Verfärbung der kosmetischen Formulierung zu verringern oder zu verhindern. EDTA bedeutet Ethylendiamintetraacetat.
Andere geeignete Chelatbildner mit dieser Eigenschaft sind Pentanatriumethylendiamintetramethylenphosphonat. Es sind aber auch Chelatbildner geeignet, die zur Gruppe der Polyamine oder alpha-Hydroxyfettsäuren gehören.

Geeignete Antioxidantien sind auch Verbindungen der Formeln A oder B 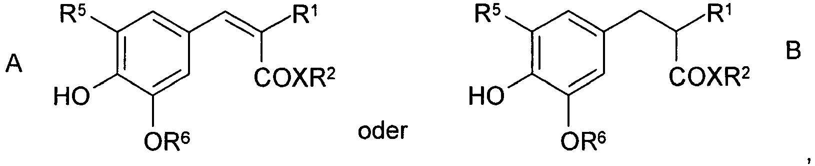 worin
- R¹: aus der Gruppe -C(O)CH₃, -CO₂R³, -C(O)NH₂ und -C(O)N(R⁴)₂ ausgewählt werden kann,
- X: O oder NH,'
- R²: lineares oder verzweigtes Alkyl mit 1 bis 30 C-Atomen,
- R³: lineares oder verzweigtes Alkyl mit 1 bis 20 C-Atomen,
- R⁴: jeweils ungabhängig voneinander H oder lineares oder verzweigtes Alkyl mit 1 bis 8 C-Atomen,
- R⁵: H, lineares oder verzweigtes Alkyl mit 1 bis 8 C-Atomen oder lineares oder verzweigtes Alkoxy mit 1 bis 8 C-Atomen und

R⁶ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex^{®} ST Liquid) und/oder 2-(4-Hydroxy-3,5-dimethoxybenzyl)-malonsäure-bis-(2-ethylhexyl)ester (z.B. RonaCare^{®} AP).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004). Derartige Antioxidantien werden mit den erfindungsgemäßen Verbindungen in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'-oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3';4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete anti-aging Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myoinositol-phosphat (DIP), cyclisches 2,3-diphosphoglycerat (cDPG), 1,1-Diglycerin-Phosphat (DGP), ß-Mannosylglycerat (Firoin), β-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Ferner können die erfindungsgemäßen Zubereitungen mindestens einen Selbstbräuner als weiteren inhaltsstoff enthalten.
Als vorteilhafte Selbstbräuner können unter anderem eingesetzt werden: 1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist das 1,3-Dihydroxyaceton, Erythrulose oder deren Kombination.

Die Zubereitungen können auch ein oder mehrere weitere hautaufhellende Wirkstoffe oder synonym Depigmentierungswirkstoffe enthalten. Hautaufhellende Wirkstoffe können prinzipiell alle dem Fachmann bekannte Wirkstoffe sein. Beispiele von Verbindungen mit hautaufhellender Aktivität sind Hydrochinon, Kojisäure, Arbutin, Aloesin oder Rucinol.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt. Ernährungsphysiologische Anwendungen orientieren sich am jeweiligen empfohlenen Vitaminbedarf.

Die beschriebenen Retinoide sind gleichzeitig auch wirksame Anti-Cellulite-Wirkstoffe. Ein ebenfalls bekannter Anti-Cellulite-Wirkstoff ist Koffein.

Die genannten Bestandteile der Zubereitung können in der üblichen Weise eingearbeitet werden, mit Hilfe von Techniken, die dem Fachmann wohl bekannt sind.

Geeignet sind Zubereitungen für eine äußerliche Anwendung, beispielsweise als Creme oder Milch (O/W, W/O, O/W/O, W/O/W), als Lotion oder Emulsion, in Form ölig-alkoholischer, ölig-wässriger oder wässrigalkoholischer Gele bzw. Lösungen auf die Haut aufgesprüht werden kann. Sie können als feste Stifte vorliegen oder als Aerosol konfektioniert sein. Für eine innerliche Anwendung sind Darreichungsformeln wie Kapseln, Dragees, Pulver, Tabletten-Lösungen oder Lösungen geeignet.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays.
Bevorzugte Hilfsstoffe stammen aus der Gruppe der Konservierungsstoffe, Stabilisatoren, Lösungsvermittler, Färbemittel, Geruchsverbesserer.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlerwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methyl-cyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Es hat sich gezeigt, dass die erfindungsgemäßen Verbindungen der Formel I, aber auch 4-Methoxycinnamoyl-6O-ascorbat, besonders bevorzugt mit polaren Ölkomponenten in Zubereitungen eingearbeitet werden können.

Besonders bevorzugte polare Ölkomponenten sind beispielsweise Arylalkylbenzoate, Dimethylisosorbid, Phthalimide, Fettsäureester, Alkylbenzoate, Triglyceride oder N,N-disubstituierte Amide.

Ein besonders bevorzugtes Arylalkylbenzoat ist 2-Phenylethylbenzoat, das kommerziell unter dem Namen X-Tend^{™} 226 der Fa. ISP angeboten wird.

Besonders bevorzugte Phthalimide sind beispielsweise n-Butylphthalimid, Isopropylphthalimid oder Gemische von Butylphthalimid und Isopropylphthalimid. Ein spezielles Gemisch wird beispielsweise unter dem Handelsprodukt Pelemol® BIP von der Fa. Phoenix Chemicals angeboten. Bevorzugte Fettsäureester sind beispielsweise Dimyristyltartrat, angeboten als COSMACOL® ETLP der Fa. Sasol, Zitronensäureester mit Alkylgruppen mit jeweils 14 oder 15 C-Atomen, angeboten als COSMACOL® ECL der Fa. Sasol, Zitronensäureester mit Alkylgruppen mit jeweils 12 oder 13 C-Atomen, angeboten als COSMACOL® ECI der Fa. Sasol, Milchsäureester mit Alkylgruppen mit jeweils 12 bis 13 C-Atomen, angeboten als COSMACOL® ELI, Tridecylsalicylat, angeboten als COSMACOL® ESI, Ester der Ethylhexansäure mit Alkylgruppen mit jeweils 12 bis 13 C-Atomen, angeboten als COSMACOL® EOI der Fa. Sasol, Maleinsäureester mit Alkylgruppen mit jeweils 12 bis 13 C-Atomen, angeboten als COSMACOL® EMI, Weinsäureester mit Alkylgruppen mit jeweils 12 bis 13 C-Atomen, angeboten als COSMACOL® ETI der Fa. Sasol.

Bevorzugte Alkylbenzoate sind Benzoesäureester mit Alkylgruppen mit jeweils 12 bis 15 C-Atomen. Handelsprodukte werden angeboten als COSMACOL® EBI der Fa. Sasol oder unter Finsolv® TN der Fa. Finetex.

Bevorzugte Triglyceride sind Triglyceride mit Fettsäuren mit jeweils 8 bis 12 C-Atomen, beispielsweise angeboten als Miglyol® 812 der Fa. Evonik.

N,N-disubsituierte Amide sind beispielsweise beschrieben in EP 1044676 oder EP 0928608. Bevorzugte N,N-disubstituierte Amide sind N-acetyl-N-butylaminopropionat, beispielsweise angeboten als IR3535 der Fa. Merck, Isopropyl N-lauroylsarcosinat, angeboten als Eldew® SL-205 der Fa. Ajimoto oder N,N-diethyltoluamid, angeboten als Deet® der Fa. Showa Denko.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemittel kann Wasser ein weiterer Bestandteil sein.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren^{®} 1200 (Henkel KGaA), Oramix^{®} NS 10 (Seppic).

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-A-43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen.

Es ist ferner von Vorteil, die Fettsäureethoxylate ausfolgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)-glyceryllaurat, Polyethylenglycol(22)glyceryllaurat, Polyethylenglycol-(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat) zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.

Bevorzugte Zubereitungen haben einen pH-Wert im Bereich von 3,5 bis 7, besonders bevorzugt im Bereich von 4,5 bis 6,5. Es hat sich als vorteilhaft herausgestellt, wenn die zur Formulierung eingesetzte Wasserphase gepuffert vorliegt. Besonders bevorzugt ist die Verwendung eines Citratpuffers (Zitronensäure/Natriumcitrat).

Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt. Die Gewichtsprozentverhältnisse der einzelnen Inhaltsstoffe in den Zubereitungen der Beispiele gehören ausdrücklich zur Offenbarung der Beschreibung und können daher als Merkmale herangezogen werden.

Die im Folgenden angeführte Beispiele für den erfindungsgemäßen Gegenstand dienen lediglich der Erläuterung und engen die vorliegende Erfindung keineswegs in irgendeiner Weise ein. Im Übrigen ist die beschriebene Erfindung im gesamten beanspruchten Bereich ausführbar. Alle Verbindungen oder Komponenten, die in den Zubereitungen verwendet werden können, sind entweder bekannt und käuflich erhältlich oder können nach bekannten Methoden synthetisiert werden. Es werden in der Regel die INCl-Namen der verwendeten Rohstoffe angegeben (die INCl-Namen werden definitionsgemäß in englischer Sprache angegeben).

### Synthesebeispiele:

### Beispiel 1: 3-(2,4-Dimethoxy-phenyl)-acrylsäure-(S)-2-((R)-3,4-dihydroxy-5-oxo-2,5-dihydro-furan-2-yl)-2-hydroxy-ethylester durch enzymatische Veresterung

### (2,4-Dimethoxyzimtsäure-6-O-ascorbat)

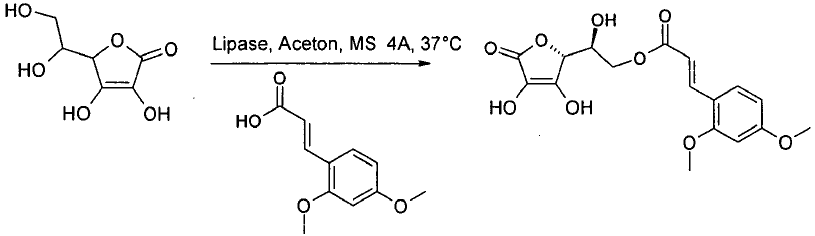

Es werden 10 g L-Ascorbinsäure (56,8 mmol; 1 Äq.) in 190 ml Aceton mit 10 g Molekularsieb 4 Å vorgelegt, 100 mg Lipase (z.B. Rhizomucor miehei, recombinant aus Aspergillus oryzae oder Candida cylindracea) und dann 29,6 g 3-(2,4-Dimethoxy-phenyl)-acrylsäure (141,9 mmol, 2,5 Äq.) zugegeben. Nach 18 Std Reaktionszeit bei 37°C wird das Molsieb abfiltriert, auf Raumtemperatur abgekühlt und das Produkt durch die langsame Zugabe von 100 ml Wasser ausgefällt. Nach Trocknen im Vakuum bei 60°C erhält man das Produkt als weißen Feststoff.

### Beispiel 2: Synthese von (E)-3-(2,4,6-Trimethoxy-phenyl)-acrylsäure (R)-2-((R)-3,4-di-hydroxy-5-oxo-2,5-dihydro-furan-2-yl)-2-hydroxy-ethylester

### (2,4,6-Trimethoxyzimtsäure-6-O-ascorbat)

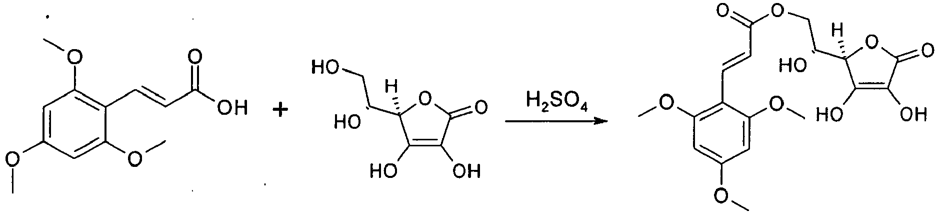

In einem mit Argon gespülten 3-Hals-Kolben werden 265 ml konz. Schwefelsäure vorgelegt und auf 0°C gekühlt. Es werden portionsweise 133,1 g (0,756 mol, 3 Äq.) Ascorbinsäure und anschließend 60 g (E)-3-(2,4,6-Trimethoxy-phenyl)-acrylsäure 0,252 mol, 1 Äq.) zugegeben. Dann werden 73,7 ml Oleum (Schwefelsäure mit 65% SO₃) zugetropft. Nach 4 Std. Reaktionszeit bei 40°C wird die Reaktionslösung auf 1000 g Eis gegossen, mit Natriumchlorid gesättigt und Ethylmethylketon extrahiert. Die vereinigten org. Phasen werden mit ges. NaCl-Lösung extrahiert, über Natriumsulfat getrocknet und das Lösungsmittel im Vakuum entfernt. Nach Kristallisation aus Ethylacetat erhält man das Produkt als hellgelben Feststoff.

¹H-NMR-Daten von 2,4,6-TMCA [(E)-3-(2,4,6-Trimethoxy-phenyl)-acrylic acid (R)-2-((R)-3,4-dihydroxy-5-oxo-2,5-dihydro-furan-2-yl)-2-hydroxy-ethyl ester]

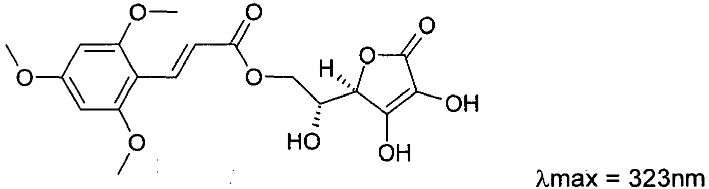

¹H-NMR (500 MHz, DMSO) ö 3.83 (s, OCH₃), 3.86 (s, 2xOCH₃), 4.03 (t, CH, J = 1.6 Hz), 4.16 (m, CH₂), 4.72 (d, CH, J = 1.3 Hz), 5.34 (br, OH), 6.28 (s, 2xCH), 6.65 (d, CH), 7.96 (d, CH), 8.39 (br, OH), 11.09 (br, OH).

### Beispiel 3: Synthese von (E)-3-(2,3,4-Trimethoxy-phenyl)-acryläure (R)-2-((R)-3,4-di-hydroxy-5-oxo-2,5-dihydro-furan-2-yl)-2-hydroxy-ethylester

### (2,3,4-Trimethoxyzimtsäure-6-O-ascorbat)

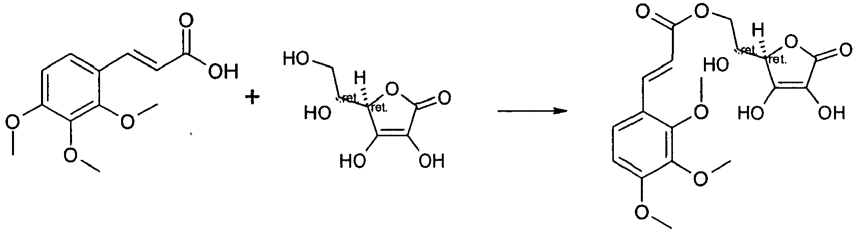

Es werden 25 g (E)-3-(2,3,4-Trimethoxy-phenyl)-acrylsäure (105 mmol; 1 Äq.) bei 25°C in 100 ml NMP gelöst, 9,1 ml Thionylchlorid (126 mmol, 1,2 Äq.) bei 0°C zugegeben und für 2 Stunden gerührt. Anschließend werden 55,4 g Ascorbinsäure (315 mmol; 3 Äq.) bei 25-28°C zugegeben und für weitere 60 min gerührt. Anschließend werden Wasser und Chloroform zugegeben, extrahiert, getrocknet und das Lösungsmittel im Vakuum entfernt. Der Rückstand wird in Toluol umkristallisiert. Man erhält das Produkt als hellbeigen Feststoff.

### Beispiel 4: Synthese von (E)-3-(2,4,5-Trimethoxy-phenyl)-acryläure (R)-2-((R)-3,4-di-hydroxy-5-oxo-2,5-dihydro-furan-2-yl)-2-hydroxy-ethylester

### (2,4,5-Trimethoxyzimtsäure-6-O-ascorbat)

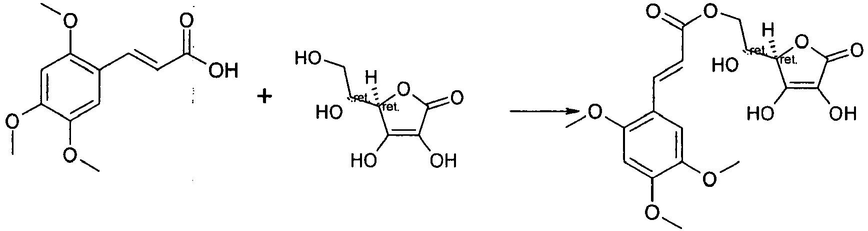

In einem mit Argon gespülten 3-Hals-Kolben werden 20 g (E)-3-(2,4,5-Trimethoxy-phenyl)-acryläure (84 mmol; 1 Äq.) in 100 ml Dimethylformamid gelöst und 11,1 g Diisopropylcarbodiimid (88,1 mmol, 1,05 Äq.) zugegeben. Nach 30 min wird die in 45 ml Dimethylformamid gelöste Ascorbinsäure (22,2 g, 126 mmol; 1,5 Äq.) zugetropft. Nach 30 min bei 0°C wird noch 6 Std. bei Raumtemperatur gerührt. Die Reaktionslösung wird zum Rückstand eingedampft, in Ethylacetat/Hexan 1:1 suspendiert, filtriert und durch Zugabe von Hexan das Produkt als hellgelben Feststoff ausgefällt (λmax = 354nm)

### Beispiel 5: Untersuchungen auf Photoisomerisierung durch Kurzzeitbestrahlung

Eine 2%ige Lösung des erfindungsgemäßen UV-Filterchromophores bzw. der Vergleichsverbindung in 1-Propanol/Pelemol BIP (8/2) wird auf die rauhe Seite eines Plexiglasobjekträgers aufgetragen (1µL/cm⁻²). Die Probe wird anschließend für 15min mit solarsimuliertem UV-Licht bestrahlt (Atlas CPS+, Betriebsstufe 765Wm⁻²). Danach werden die PMMA-Plates mit 50 ml Isopropanol abgespült und per UV-VIS Spektrometer vermessen. Im Vergleich zum Dunkelwert wird die Absorptionsabnahme am Absorptionsmaximum bestimmt.

### Untersuchte Verbindungen:

A) (2,4-Dimethoxyzimtsäure-6-O-ascorbat)
B) (3,4-Dimethoxyzimtsäure-6-O-ascorbat) zum Vergleich.

### Ergebnis:

Verbindung A) zeigt eine Absorptionsabnahme von 26% wohingegen Verbindung B) eine Absorptionsabnahme von 40% aufweist. Damit zeigt das ortho-substituierte Zimtsäurederivat eine geringere Absorptionsabnahme und das Ergebnis belegt die geringere Bildung des cis-isomeren.

### Beispiel 6: Untersuchungen auf Photoisomerisierung (reversible Photoisomerisierung) durch Kurzzeitbestrahlung

Eine 2%ige Lösung des erfindungsgemäßen UV-Filterchromophores bzw. der Vergleichsverbindung in 1-Propanol/Pelemol BIP (8/2) wird auf die rauhe Seite eines Plexiglasobjekträgers aufgetragen (1µL/cm⁻²). Die Probe wird anschließend für 15min mit solarsimuliertem UV-Licht bestrahlt (Atlas CPS+, Betriebsstufe 765Wm⁻²). Danach werden die PMMA-Plates mit 50 ml Isopropanol abgespült und per UV-VIS Spektrometer vermessen. Im Vergleich zum Dunkelwert wird die Absorptionsabnahme am Absorptionsmaximum bestimmt.

### Untersuchte Verbindungen:

A) 2,4,6-Trimethoxyzimtsäure-6-O-ascorbat
B) 4-Methoxyzimtsäureethylhexylester zum Vergleich.

### Ergebnis:

Verbindung A) zeigt eine Absorptionsabnahme von 20% wohingegen Verbindung B) eine Absorptionsabnahme von 37% aufweist. Damit zeigt das ortho-substituierte Zimtsäurederivat eine geringere Absorptionsabnahme als die Vergleichskomponente.

### Beispiele für Zubereitungen:

### Beispiel 1: Allgemeine Formulierungshinweise:

Beschriebene Ascorbate von Zimtsäurederivaten können sowohl in die Ölphase von Zubereitungen, wie z.B. Emulsionen eingearbeitet werden als auch in deren Wasserphase. Bevorzugt ist auch eine kombinierte Einarbeitung in Öl- und Wasserphase möglich, wodurch hinsichtlich der Gesamtwirksamkeit der Zubereitung Synergieeffekte zum tragen kommen. Insbesondere bei Einarbeitung in die Wasserphase kosmetischer Emulsionen ist bzgl. der Gesamt-UV-Schutzwirkung der Formulierung ein Synergieeffekt zwischen dem (den) wasserlöslichen UV-Filter(n) und weiteren enthaltenen öllöslichen UV-Filtern zu erzielen. Zur Einarbeitung in Öl- oder Wasserphase ist die Verwendung von Löslichkeitsvermittlern von Vorteil. Dabei ist z.B. der Zusatz von Alkoholanteilen vorteilhaft (e.g. Ethanol, Isopropanol). Der pH-Wert der Formulierung sollte bevorzugt zwischen pH=3 und pH=6 liegen, um durch saure Umgebung in der Formulierung eine zufriedenstellende Stabilität des Ascorbats zu erzielen. Besonders bevorzugt ist es z.B. den pH-Wert der Wasserphase mit Citratpuffer auf pH=5 zu puffern, da dies dem natürlichen pH-Wert der Haut entspricht. Generell können die beschriebenen Ascorbate derart in mindestens eine lipophile oder hydrophile Phase einer Zubereitung eingearbeitet werden, dass entweder eine klare Lösung vorliegt oder die Substanzen dispergiert vorliegen.

### Beispiel 2: W/O Emulsion - Zahlenangaben in Gew.-%

| | **a** | **b** | **c** | **d** | **e** |
|---|---|---|---|---|---|
| Cetyl PEG/PPG-10/1 dimethicone (Abil EM 90) | 3 | 3 | 3 | 3 | 3 |
| Polyglyceryl-4 isostearate (Isolan GI 34) | 1,5 | 1,5 | 1,5 | 1,5 | 1,5 |
| Butylphthalimide isopropylghthalimide (Pelemol^{®} BIP) | 5 | 5 | 5 | 5 | 5 |
| Dimethyl isosorbide (Arlasolve DMI) | 5 | 5 | 5 | 5 | 5 |
| 2,4-dimethoxyzimtsäure-6-O-ascorbat | 2 | 1 | 0,5 | | |
| 2,4,6-trimethoxyzimtsäure-6-O-ascorbat | | | | 1 | 1,5 |
| 2,3,4-trimethoxyzimtsäure-6-O-ascorbat | 1 | 1,5 | 0,5 | | |
| 2,4,5-trimethoxyzimtsäure-6-O-ascorbat | | | | 1 | 1 |
| Uvinul^{®} A Plus (DHHB) | | 1 | 1 | 1 | |
| Ascorbinsäure | | | 0,37 | 1 | 3 |
| Mineral Oil | 8 | 8 | 8 | 8 | 8 |
| Ethylhexyl stearate (Tegosoft^{®} OS) | 5 | 5 | 5 | 5 | 5 |
| Cyclomethicone (and) Aluminium/Magnesium Hydroxide Stearate (Gilugel SIL 5) | 5 | 5 | 5 | 5 | 5 |
| Preservative | 1 | 1 | 1 | 1 | 1 |
| Water | Ad 100 | Ad 100 | Ad 100 | Ad 100 | Ad 100 |
| 4-methoxyzimtsäure-6-O-ascorbat | 1 | 2 | 3 | 4 | 5 |
| NaCl | 0,5 | 0,5 | 0,5 | 0,5 | 0,5 |
| EDTA | 0,1 | 0,1 | 0,1 | 0,1 | 0,1 |
| Citronensäure q.S. | | | | | |

Herstellung: Pelemol^{®} BIP, Arlasolv DMI und Emulgatoren werden vorgelegt. 2,4-dimethoxyzimtsäure-6-O-ascorbat, 2,4,6-trimethoxyzimtsäure-6-O-ascorbat, 2,3,4-trimethoxyzimtsäure-6-O-ascorbat, 2,4,5-trimethoxyzimtsäure-6-O-ascorbat und Uvinul^{®} A Plus werden darin gelöst. Die restlichen Bestandteile der Ölphase werden zugegeben und homogen vermischt. Unter Rühren wird die mit Citronensäure auf pH=4-5 eingestellte Wasserphase enthaltend 4-methoxyzimtsäure-6-O-ascorbat einemulgiert. Anschließend wird homogenisiert. Die Emulsionen können unter schonenden Bedingungen bei Raumtemperatur hergestellt werden. Durch Erhöhung des Gehaltes an Ascorbinsäure können enthaltene Zimtsäureascorbate stabilisiert werden. Idealerweise wird die Herstellung unter Inertisierung (Ausschluß von Sauerstoff hergestellt).

### Beispiel 3: Wasserfestes Sonnenschutzspray - Zahlenangaben in Gew.-%

| A | | | |
|---|---|---|---|
| 2,4-dimethoxyzimtsäure-6-O-ascorbat | 1 | 1 | 2 |
| 4-methoxyzimtsäure-6-O-ascorbat | 0,5 | 0,5 | 0,5 |
| Diethylhexyl Syringylidenemalonate, Caprylic/Capric Triglyceride (Oxynex^{®} ST Liquid) | | 0,5 | |
| 2-(4-Hydroxy-3,5-dimethoxybenzyl)-malonsäure-bis-(2-ethylhexyl)ester RonaCare^{®} AP | | 2 | |
| Ascorbyl Palmitate | | | 1 |
| Cyprylic/capric Triglyceride (Miglyol 812 N) | 7 | 7 | 7 |
| Butylphthalimide isopropylphthalimide | 9 | 9 | 9 |
| (Pelemol^{®} BIP) | | | |
| C12-15 alkyl benzoate (Tegosoft^{®} TN) | 10 | 10 | 10 |
| Phenethyl benzoate (X-Tend 226) | 5 | 5 | 5 |
| RonaCare^{®} Tocopherolacetat | 1 | 1 | 1 |

| B | | | |
|---|---|---|---|
| Cyclopentasiloxane (Dow Corning 245) | 43,8 | 41,3 | 41,8 |
| 4-methoxyzimtsäure-6-O-ascorbat | 0,5 | 0,5 | 0,5 |
| Phenyltrimethicone (Dow Corninq 556) | 2 | 2 | 2 |
| Cyclopentasiloxane, dimethiconol Dow Corning 1501 Fluid | 20 | 20 | 20 |
| Parfümöl (q.s.) | 0,2 | 0,2 | 0,2 |

Herstellung: Die Komponenten der Phase A werden bei Raumtemperatur zusammengefügt und gerührt bis eine klare Lösung oder homogene Disperison vorliegt. Anschließend wird Phase B gemischt und unter Rühren zu Phase B gegeben. Man rührt weiter, bis letztlich das homogene Produkt vorliegt. Zu Zugabe von Antioxidantien wie Oxynex^{®} ST Liquid, RonaCare^{®} AP oder Ascorbylpalmitat kann die Stabilität der erfindungsgemäßen Substanzen erhöht werden.

### Beispiel 4: Pump Haarspray - Zahlenangaben in Gew.-%

| A | | | |
|---|---|---|---|
| 2,4-dimethoxyzimtsäure-6-O-ascorbat | 1 | 1 | 4 |
| 2,4,6-trimethoxyzimtsäure-6-O-ascorbat | 1 | | |
| 2,3,4-trimethoxyzimtsäure-6-O-ascorbat | | 1 | |
| 2,4,5-trimethoxyzimtsäure-6-O-ascorbat | | | 1 |
| 4-methoxyzimtsäure-6-O-ascorbat | 2 | 2 | 2 |
| Ethanol 96% reinst | Ad 100 | Ad 100 | Ad 100 |
| PVP/VA copolymer | 6 | 6 | 6 |
| PVP/VA W 735 | | | |
| B | | | |
| Diethylhexyl Syringylidenemalonate, Caprylic/Capric Triglyceride | 0,06 | 0,25 | 0,50 |
| (Oxynex^{®} ST Liquid) | | | |
| PEG-75 Lanolin BHT | 0,2 | 0,2 | 0,2 |
| (Solan E - Low Dioxane) | | | |
| Parfum | 0,1 | 0,1 | 0,1 |
| (Frag 280853 Green Activating) | | | |
| C | | | |
| Wasser, demineralisiert | 13 | 13 | 13 |
| Titriplex III | 0,1 | 0,1 | 0,1 |
| PEG-12 dimethicone | 0,5 | 0,5 | 0,5 |
| Dow Corning 193 Fluid | | | |
| 0,1% D&C Red No 33 (CI 17200) in Wasser | 0,2 | 0,2 | 0,2 |
| PEG-40 Hydrogenated Castor Oil (Cremophor RH 410) | 1 | 1 | 1 |

Herstellung: Phase A vorlösen, bis eine klare Lösung vorliegt. Unter Rühren Phase B zu Phase A geben. Phase C vormischen und zum Rest geben, rühren, bis eine homogene Mischung entstanden ist.

### Beispiel 5: W/O-Emulsionen -Zahlenangaben in Gew.-%

| **Emulsion** | **A** | **B** | **C** | **D** | **E** | **F** |
|---|---|---|---|---|---|---|
| Polyglyceryl-2-Dipolyhydroxystearat | 3 | 5 | 3 | | | |
| PEG-30 Dipolyhydroxystearat | | | 2 | 3 | 4 | 5 |
| Natrium-Stärke Octenylsuccinat | 0,5 | 0,4 | | 0,3 | | 1 |
| Glycin | 0,3 | 0,3 | 0,5 | 0,4 | | |
| Alkohol | | 5 | 2 | 5 | 4 | |
| Magnesiumsulfat | 0,2 | 0,3 | 0,3 | 0,4 | 0,5 | 0,2 |
| C₁₂₋₁₅ Alkyl Benzoat | 5 | 3 | | | 5 | |
| C₁₂₋₁₃ Alkyl Tartrat | | 2 | | | | |
| Butylenglycol Dicaprylat/Dicaprat | 5 | | | | 3 | 3 |
| Dicaprylyl Ether | | | | | 2 | |
| Mineralöl | | 4 | | 6 | | 8 |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Propylenglykol | | | 1 | | 5 | 3 |
| Glycerin | 3 | 5 | 7 | 10 | 3 | 3 |
| C18-38 Säuretriglyceride | 0,5 | | 1 | | 1 | |
| Titandioxid | 5 | 6 | 4 | | | 4 |
| Zinkoxid | 5 | | | | | |
| Bis-Ethylhexyloxyphenol Methoxyphenyltriazin | | 3 | 3 | 2 | | |
| Ethylhexyltriazon | | 4,5 | 3 | | 3 | |
| 2,4-dimethoxyzimtsäure-6-O-ascorbat | 2 | 0,5 | 1 | 1 | 3 | 1,5 |
| 4-methoxyzimtsäure-6-O-ascorbat | 0,5 | 1,5 | 1 | 2 | 0,5 | 1,5 |
| Diethylhexylbutamidotriazon | | | 1,5 | 4 | | |
| Butyl Methoxydibenzoylmethan | 2 | 3 | 4 | | 1 | 3 |
| Uvinul^{®} A Plus | | | | 4 | 2 | |
| Ethylhexylmethoxycinnamat | | | | | 7 | 5 |
| 2,4-dimethoxyzimtsäure-6-O-ascorbat an Gelatine gekoppelt | 1,5 | 5,5 | | 8 | 4,5 | 7,5 |
| Benzotriazol an Gelatine gekoppelt | 4 | | 6 | | | |
| Taurin | 0,1 | | | 0,5 | 0,2 | |
| Vitamin E Acetat | 0,2 | 02 | | 0,3 | 0,1 | 0,5 |
| Na₂H₂EDTA | 0,1 | 0,1 | 0,2 | 0,2 | 0,2 | 0,5 |
| C8-C16 Alkylpolyglycosid | 1 | | | | | |
| Parfüm, Konservierungsmittel | q.s. | q.s | q.s | q.s | qs. | qs. |
| Farbstoffe, usw. | q.s. | q.s. | q.s. | q.s | q.s. | q.s. |
| Natriumhydroxid | q.s. | q.s. | q.s. | q.s | q.s. | q.s. |
| Wasser | ad 100,0 | ad 100,0 | ad 100,0 | ad 100,0 | ad 100,0 | ad 100,0 |

### Beispiel 6: Haarpflegeformulierung - Zahlenangaben in Gew.-%

| **Gehalt in g Komponente per 100 g Formulierung** | | | | | | |
|---|---|---|---|---|---|---|
| **Komponente** | **A** | **B** | **C** | **D** | **E** | **F** |
| Disodium EDTA | 0.1 | 0.1 | 0.1 | 0.1 | 0.1 | 0.1 |
| Oxynex^{®}ST | 2 | 2 | 2 | 2 | 2 | 2 |
| 4-dimethoxyzimtsäure-6-O-ascorbat | 0,1 | 0,25 | 0,5 | 1,5 | 2 | 4 |
| 2,4-dimethoxyzimtsäure-6-O-ascorbat | 4 | 2 | 1,5 | 0,5 | 0,25 | 0,1 |
| 2,4,6-trimethoxyzimtsäure-6-O-ascorbat | 0,5 | 1 | 1,5 | 2 | 2,5 | 3 |
| Hexamidine diisethionate | 0.1 | 0 | 0 | 0 | 0 | 0 |
| Tetrahydrocurcumin | 0 | 0.5 | 0 | 0 | 0 | 0 |
| Glycyrrhetinic acid | 0 | 0 | 0.3 | 0 | 0 | 0 |
| Thiotaine®¹ | 0 | 0 | 0 | 5 | 0 | 0 |
| N-undecylenoyl-L-phenylalanine | 0 | 0 | 0 | 0 | 1 | 0 |
| N-acetyl glucosamine | 0 | 0 | 0 | 0 | 0 | 2 |
| Niacinamide | 5 | 5 | 5 | 5 | 5 | 5 |
| Citric acid | 0.015 | 0 | 0 | 0 | 0 | 0 |
| Isohexadecane | 3 | 3 | 3 | 3 | 3 | 3 |
| Isopropyl isostearate | 1.33 | 1.33 | 1.33 | 1.33 | 1.33 | 1.33 |
| Isopropyl N-laurosylsarcosinate | 0 | 0 | 5 | 0 | 0 | 0 |
| Sucrose polycottonseedate | 0.67 | 0.67 | 0.67 | 0.67 | 0.67 | 0.67 |
| Polymethylsilsesquioxane | 0.25 | 0.25 | 0.25 | 0.25 | 0.25 | 0.25 |
| Cetearyl glucoside + cetearyl alcohol | 0.2 | 0.2 | 0.2 | 0.2 | 0.2 | 0.2 |
| Behenyl alcohol | 0.4 | 0.4 | 0.4 | 0.4 | 0.4 | 0.4 |
| Ethylparaben | 0.2 | 0.2 | 0.2 | 0.2 | 0.2 | 0.2 |
| Propylparaben | 0.1 | 0.1 | 0.1 | 0.1 | 0.1 | 0.1 |
| Cetyl alcohol | 0.32 | 0.32 | 0.32 | 0.32 | 0.32 | 0.32 |
| Stearyl alcohol | 0.48 | 0.48 | 0.48 | 0.48 | 0.48 | 0.48 |
| Tocopheryl acetate | 0.5 | 0.5 | 0.5 | 0.5 | 0.5 | 0.5 |
| PEG-100 stearate | 0.1 | 0.1 | 0.1 | 0.1 | 0.1 | 0.1 |
| Glycerin | 7 | 7 | 7 | 7 | 7 | 7 |
| Titanium dioxide | 0.604 | 0.604 | 0.604 | 0.604 | 0.604 | 0.604 |
| Polyacrylamide + C13-14 isoparaffin + laureth-7 | 3 | 2 | 2 | 2 | 2 | 2 |
| Panthenol | 1 | 1 | 1 | 1 | 1 | 1 |
| Benzyl alcohol | 0.4 | 0.4 | 0.4 | 0.4 | 0.4 | 0.4 |
| Dimethicone + dimethiconol | 2 | 2 | 2 | 2 | 2 | 2 |
| Water (to 100 g) | to 100 | to 100 | to 100 | to 100 | to 100 | to 100 |
| TOTAL | 100 | 100 | 100 | 100 | 100 | 100 |

### Beispiel 7: Haarpflegeformulierung - Zahlenangaben in Gew.-%

| **Gehalt in g Komponente per 100 g Formulierung** | | | |
|---|---|---|---|
| **Komponente** | **G** | **H** | **I** |
| Disodium EDTA | 0.1 | 0.1 | 0.1 |
| Oxynex^{®} ST | 2 | 2 | 2 |
| 2,3,4-trimethoxyzimtsäure-6-O-ascorbat | 0.5 | 3.5 | 1.5 |
| 4-methoxyzimtsäure-6-O-ascorbat | 1.5 | 0.5 | 2 |
| Cetyl pyridinium chlorid | 0.2 | 0 | 0 |
| Pitera^{®} | 0 | 10 | 0 |
| Ascorbyl glycoside | 0 | 0 | 2 |
| Niacinamide | 3.5 | 5 | 4 |
| Polyquaternium 37 | 0 | 0 | 0 |
| Isohexadecane | 3 | 2.5 | 2 |
| Isopropyl isostearate | 1.33 | 1.33 | 1.33 |
| Sucrose polycottonseedate | 0.67 | 0.67 | 0.67 |
| Polymethylsilsesquioxane | 0.25 | 0.25 | 0.25 |
| Cetearyl glucoside + cetearyl alcohol | 0.2 | 0.2 | 0.2 |
| Behenyl alcohol | 0.4 | 0.4 | 0.4 |
| Ethylparaben | 0.2 | 0.2 | 0.2 |
| Propylparaben | 0.1 | 0.1 | 0.1 |
| Cetyl alcohol | 0.32 | 0.32 | 0.32 |
| Stearyl alcohol | 0.48 | 0.48 | 0.48 |
| Tocopheryl acetate | 0.5 | 0.5 | 0.5 |
| PEG-100 stearate | 0.1 | 0.1 | 0.1 |
| Glycerin | 7 | 7 | 7 |
| Titanium dioxide | 0.604 | 0.604 | 0.604 |
| Polyacrylamide + C13-14 isoparaffin + laureth-7 | 2 | 2 | 2 |
| Panthenol | 1 | 1 | 1 |
| Benzyl alcohol | 0.4 | 0.4 | 0.4 |
| Dimethicone + dimethiconol | 2 | 2 | 2 |
| Water (to 100 g) | to 100 | to 100 | to 100 |
| TOTAL | 100 | 100 | 100 |

### Beispiel 8: O/W-Emulsionen - Zahlenangaben in Gew.-%

| **Emulsion** | **A** | **B** | **C** | **D** | **E** | **F** |
|---|---|---|---|---|---|---|
| Glyceryl Stearat Citrat | 2,5 | 2 | 3 | | | |
| Sorbitanstearat | 0,5 | | | 2 | 1,5 | 2 |
| Polyglyceryl-3 Methylglycose Distearat | | | | 2,5 | 3 | 3 |
| Polyglyceryl-2 Dipolyhydroxystearat | | 0,8 | | | | 0,5 |
| Cetearylalkohol | | | | 1 | | |
| Stearylalkohol | 2 | | | | | 2 |
| Cetylalkohol | | 1 | | | 3 | |
| Acrylates/C₁₀₋₃₀ Alkyl Acrylat Crosspolymer | | 0,2 | | | 0,1 | |
| Carbomer | | 0,2 | 0,3 | 0,2 | | |
| Xanthan Gum | 0,4 | | 0,2 | 0,2 | 0,3 | 0,4 |
| C₁₂₋₁₅ Alkyl Benzoat | 5 | 3 | | | 5 | |
| C₁₂₋₁₃ Alkyl Tartrat | | 2 | | | | |
| Butylenglycol Dicaprylat/Dicaprat | 5 | | | | 3 | 3 |
| Dicaprylyl Ether | | | | | 2 | |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Propylengykol | | | 1 | | 5 | 3 |
| Glycerin | 3 | 5 | 7 | 10 | 3 | 3 |
| C18-C38 Säuretriglyceride | 0,5 | | 1 | | 1 | |
| Titandioxid | 5 | | | 2 | | |
| 2,2'-Methylen-bis-(6-(2H-benzotriazol-2-yl)-(1,1,3,3-tetramethylbutyl)phenol) | 2,5 | | | | | |
| 2,4,6-Tris-(biphenyl)-1,3,5-triazin | | 2 | | | | |
| Merocyanin an Gelatine gekoppelt | 6 | | 6 | | 10 | 3 |
| Benzotriazol an Gelatine gekoppelt | | 5 | | 10 | | 3 |
| C8-C16 Alkylpolyglycosid | 1 | 0,6 | | | | |
| UVASorb^{®} K2A | | | 2 | | | |
| Uvinul^{®} A Plus | 2 | | | | | 1 |
| Homosalat | | 5 | | 1 | | |
| Phenylbenzimidazol Sulfonsäure | | | 2 | | | 1 |
| Benzophenon-3 | 2 | | | | 2 | |
| Octylsalicylat | 5 | 5 | | 2 | | |
| Octocrylen | 2 | | | | 3 | 1 |
| 2,4-dimethoxyzimtsäure-6-O-ascorbat | 1,0 | 2,0 | 0,25 | 1,0 | 2,0 | 0,5 |
| 4-methoxyzimtsäure-6-O-ascorbat | 1,0 | 2,0 | 0,25 | 1,0 | 1,5 | 3,0 |
| Bis-Ethylhexyloxyphenol Methoxyphenyltriazin | | 3 | 2 | 1 | | |
| Parsol^{®} SLX | | | 3 | | | |
| Dihydroxyacetat | | | | | 4 | |
| Taurin | 0,1 | | | 0,5 | 0,2 | |
| 8-Hexadecen-1,16-dicarbonsäure | | 0,2 | | | | |
| Vitamin E Acetat | 0,2 | 0,2 | | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100, 0 |

### Beispiel 9: O/W-Emulsionen - Zahlenangaben in Gew.-%

| **Emulsion** | **G** | **H** | **I** | **K** | **L** | **M** |
|---|---|---|---|---|---|---|
| Ceteareth-20 | 1 | 1,5 | 1 | | | |
| Sorbitanstearat | | | 0,5 | | 0,5 | |
| Glyceryl Stearat SE | | | | 1 | 1 | 1,5 |
| Emulgade F^{®} | | | | 2,5 | 2,5 | 3 |
| Cetearylalkohol | | | | 1 | | |
| Stearylalkohol | | | | | 1,5 | |
| Cetylalkohol | | | 0,5 | | | 2 |
| Acrylates/C₁₀₋₃₀ Alkyl Acrylat Crosspolymer | 0,2 | 0,4 | 0,3 | 0,1 | | |
| Carbomer | | | | | 0,3 | |
| Xanthan Gum | | | | 0,4 | | 0,4 |
| C₁₂₋₁₅ Alkyl Benzoat | 5 | 3 | | | 5 | |
| 2-Phenylbenzoat | | 2 | | | | |
| Butylenglycol Dicaprylat/Dicaprat | 5 | | | | 3 | 2 |
| Dicaprylyl Ether | | | | | 2 | |
| Diethylhexylnaphthalat | 2 | | | | | |
| Dicaprylcaprat | | 2 | | | 2 | 2 |
| Cyclomethicon | 5 | | 5 | 10 | | |
| Isohexadecan | | | | 5 | | |
| Mineralöl | | 1 | | | | |
| Propylenglykol | | | 4 | | | |
| Glycerin | 5 | 7 | 3 | 5 | 6 | 8 |
| C18-38 Säuretriglyceride | 0,5 | | 1 | | 1 | |
| Titandioxid | 5 | | 3 | 2 | | |
| NeoHeliopan^{®} AP | | 2 | | | 1 | 1 |
| Phenylbenzimidazol Sulfonsäure | 1 | | | 1 | 2 | 1 |
| Ethylhexylmethoxycinnamat | 5 | | 4 | 4 | | |
| Ethylhexyltriazon | | 2 | | 1 | | |
| Diethylhexylbutamidotriazan | 1 | | | | | |
| Butyl Methoxydibenzoylmethan | 2,5 | | 2 | 2 | | 1 |
| Bis-Ethylhexyloxyphenol Methoxyphenyltriazin | 2 | | | | | |
| 4-Methylbenzyliden Camphor | 3 | | | | | |
| Parsol® SLX | | | | | 2 | |
| 2,3,4-trimethoxyzimtsäure-6-O-ascorbat | 0,5 | 1,0 | 2,0 | 0,25 | 0,75 | 1,5 |
| 2,4,6-trimethoxyzimtsäure-6-O-ascorbat | 0,5 | 1,0 | 2,0 | 0,25 | 0,75 | 1,5 |
| 4-methoxyzimtsäure-6-O-ascorbat | 0,5 | 1,0 | 2,0 | 1,5 | 1,5 | 3,0 |
| Kreatinin | 0,1 | 0,01 | 0,05 | | | |
| Kreatin | 0,5 | 0,2 | 0,1 | | | |
| Licorice Extrakt/Licochalkon | | | | 0,5 | | |
| Vitamin E Acetat | 0,2 | | | 0,5 | 0,5 | 0,5 |
| Tapioka Stärke | | 3 | | | 2 | |
| Na₂H₂EDTA | 0,1 | | 0,2 | | | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

### Beispiel 10: O/W-Emulsionen - Zahlenangaben in Gew.-%

| **Emulsion** | **N** | **O** | **P** | **Q** | **R** | **S** |
|---|---|---|---|---|---|---|
| Glycerylstearat SE | | 2 | | 2 | | |
| Glycerylstearat | 2 | | 2 | | | |
| PEG-40 Stearat | | | 2 | | 1 | |
| PEG-10 Stearat | | | | 2,5 | 1 | |
| Ceteareth-20 | | | | | | 2,6 |
| Natrium Cetyl Phosphate | | | | | 2 | |
| Glyceryl Stearat, Ceteareth-12, Ceteareth-20, Cetearyl Alcohol, Cetyl Palmitat | | | | | | 5,4 |
| Stearinsäure | 3 | 2 | | | 2 | |
| Stearylalkohol | | 2 | 2 | | | |
| Stearylalkohol | 0,5 | | 2 | | | |
| Cetylalkohol | 3 | | | 2 | | |
| Acrylates/C₁₀₋₃₀ Alkyl Acrylat Crosspolymer | | | 0,2 | | 0,4 | |
| Carbomer | | 0,3 | | 0,3 | 0,3 | |
| Xanthan Gum | | 0,3 | 0,4 | | | |
| C₁₂₋₁₅ Alkyl Benzoat | 5 | | | | 5 | 3 |
| 2-Phenylbenzoat | 5 | | | | | |
| Butylenglycol Dicaprylat/Dicaprat | | 5 | | 4 | | 3 |
| Dicaprylyl Ether | | 2 | | | 3 | |
| Diethylhexylnaphthalat | 3 | | | | | |
| Cyclomethicon | 2 | | 10 | 2 | | |
| Isohexadecan | | | | 2 | 3 | |
| Mineralöl | | | | | 3 | |
| Propandiol | | 3 | | 5 | | |
| Glycerin | 3 | 5 | 10 | 7 | 4 | 5 |
| Titandioxid | 2 | 4 | | | | |
| Zinkoxid | | | | | 2 | |
| Drometrizole Trisiloxane | | | | | 3 | |
| Ethylhexylmethoxycinnamat | | 6 | 5 | | | |
| Phenylbenzimidazol Sulfonsäure | | 0,5 | 2 | | 1 | |
| Homosalat | 5 | | | 7 | | |
| Butyl Methoxydibenzoylmethan | | 3 | | | | |
| Bis-Ethylhexyloxyphenol Methoxyphenyltriazin | | 2 | 3 | | | |
| Octylsalicylat | | | | 5 | | |
| Octocrylen | | | | | 3 | |
| 2,4,5-trimethoxyzimtsäure-6-O-ascorbat | 0,25 | 1,5 | 0,5 | 2,5 | 1,0 | 3,0 |
| 4-methoxyzimtsäure-6-O-ascorbat | 0,5 | 1,0 | 1,5 | 2,5 | 3,0 | 3,0 |
| Parsol^{®} SLX | 4 | | | | | 5 |
| PVP Hexadecen Copolymer | 0,5 | | 1 | | 0,8 | |
| Coenzym Q 10 | 0,2 | 0,02 | | 0,3 | | |
| Vitamin E Acetat | 0,2 | | 0,3 | | 0,8 | 0,5 |
| Na₂H₂EDTA | 0,1 | | | | | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

### Beispiel 11: Hydrodisperionen (Lotionen und Sprays) - Zahlenangaben in Gew.-%

| | **A** | **B** | **C** | **D** | **E** | **F** |
|---|---|---|---|---|---|---|
| Glyceryl Stearat Citrat | | 0,4 | | | | |
| Cetyl Alkohol | | | | | 2 | |
| Natrium Carbomer | | | | | 0,3 | |
| Acrylates/C₁₀₋₃₀ Alkyl Acrylate Crosspolymer | 0,3 | | 0,3 | 0,4 | 0,1 | 0,1 |
| Cetsareth-20 | | | 1 | | | |
| Xanthan Gummi | | | | 0,15 | | 0,5 |
| Dimethicon / Vinyl Dimethicon Crosspolymer | | | | 5 | | 3 |
| UVASorb^{®} K2A | | | | | 3,5 | |
| Uvinul^{®}A Plus | 0,25 | | | 0,5 | 2 | 1,5 |
| Butyl Methoxydibenzoylmethan | 1,2 | | 3,5 | | | |
| Bis-Ethylhexyloxyphenol Methoxyphenyl Triazin | 2 | 2 | | 0,25 | | |
| Terephthaliden Dicampher Sulfonsäure | | | | | | 0,5 |
| Dinatrium Phenyl Dibenzimidazol Tetrasulfonat | | | | | | 1 |
| Phenylbenzimidazol Sulfonsäure | | | 2 | | | |
| Ethylhexyl Methoxycinnamat | 5 | | 7 | | 5 | 8 |
| Diethylhexyl Butamido Triazon | | | 2 | 2 | | |
| Ethylhexyl Triazon | 4 | 3 | | | 4 | |
| Octocrylen | | | | 10 | | 2,5 |
| 2,4-dimethoxyzimtsäure-6-O-ascorbat | 0,25 | 1,5 | 0,5 | 2,5 | 1 | 5 |
| 4-methoxyzimtsäure-6-O-ascorbat | 1 | 2 | 1 | 2 | 1 | 2 |
| C₁₂₋₁₅ Alkyl Benzoat | 2 | | 2,5 | | | |
| Phenethyl Benzoat | 4 | | | 7,5 | | 5 |
| C₁₈₋₃₆ Triglycerid Fettsäure | | | 1 | | | |
| Butylenglycol Dicaprylat/Dicaprat | | | | | 6 | |
| Dicaprylyl Carbonat | | 3 | | | | |
| Dicaprylylether | | 2 | | | | |
| Cyclomethicon | | | | 1,5 | | |
| Lanolin | | | | | 0,35 | |
| PVP Hexadecen Copolymer | 0,5 | | 0,5 | | 0,5 | 1 |
| Ethylhexyloxyglycerin | | 0,75 | | 1 | | 0,5 |
| Glycerin | 10 | 5 | 5 | | 5 | 15 |
| Butylenglycol | | 7 | | | | |
| Glycin Soja | | | | 1 | | |
| Vitamin E Acetat | 0,5 | 0,25 | 05 | 0,25 | 0,75 | 1 |
| α-Glycosylrutin | | | | | 0,25 | |
| Trinatrium EDTA | | 1 | 1 | 0,1 | 0,2 | |
| Idopropinylbutylcarbamat | 0,2 | 0,1 | | | | 0,15 |
| Methylparaben | 0,5 | | 0,2 | | 0,15 | |
| Phenoxyethanol | 0,5 | 0,4 | 0,4 | | 1 | 0,6 |
| Ethanol | 3 | 10 | 4 | 3,5 | | 1 |
| Parfüm, Farbstoffe | q.s. | q.s. | q.s. | qs. | q.s. | q.s. |
| Wasser | ad 100 | ad 100 | ad 100 | ad 100 | ad 100 | ad 100 |
| Neutralisationsmittel (Natriumhydroxid, Kaliumhydroxid) | qs | qs | qs | qs | qs | qs |

### Beispiel 12: wässrige und wässrig/alkoholische Formulierungen - Zahlenangaben in Gew.-%

| | **A** | **E** | **C** | **D** | **E** | **F** |
|---|---|---|---|---|---|---|
| Ethanol | 50 | 5 | 2 | 40 | 15 | |
| Hydroxyethylcellulose | 0.5 | | | | | |
| Acrylates/C10-30 Alkyl Acrylate Crosspolymer | | | | 0,3 | 0,6 | |
| Cocoatnidopropylbetain | | | 0,3 | | | |
| UVASorb® K2A | | | | | 2 | |
| Uvinul® APlus | 5 | | | | | |
| Butyl Methoxydibenzoylmethan | 0,5 | | | 3 | | |
| Dinatrium Phenyl Dibenzimidazol Tetrasulfonat | | 2 | 1 | | | |
| Phenylbenzimidazol Sulfonsäure | | 5 | 3 | | 2 | 4 |
| Ethylhexyl Methoxycinnamat | 10 | | | | 3 | |
| Diethylhexyl Butamido Triazon | | | | 3 | | |
| Ethylhexyl Triazon | | | | | 2 | |
| Octocrylen | | | | 5 | | |
| 4-methoxyzimtsäure-6-O-ascorbat | 2,5 | 0,75 | 1,5 | 3 | 3,5 | 4 |
| 2,4,6-trimethoxyzimtsäure-6-O-ascorbat | 0,25 | 0,50 | 0,75 | 1 | 1,25 | 1,5 |
| C₁₂₋₁₅ Alkyl Benzoat | | | | 3 | | |
| C18-36 Triglycerid Fettsäure | | | | 1 | | |
| Butylenglycol Dicaprylat/Dicaprat | 2 | | | | | |
| C12-13 Alkyl Tartrat | | | | | 5 | |
| Cyclomethicon | 4 | | | 2 | | |
| Insekt Repellent^{®} 3535 | | | | 5 | | |
| Dimethicon | | | | | 3 | |
| PVP Hexadecen Copolymer | | 0,5 | | 1 | | 0.5 |
| Ethylhexyloxyglycerin | | 0,5 | | | | |
| Glycerin | 5 | 7 | 3 | 8 | | S |
| Butylenglycol | | | 5 | | 5 | |
| Metylpropandiol | | | | 4 | | |
| Vitamin E Acetat | | 0,3 | 0,2 | 0,5 | | |
| Panthenol | 0.5 | | 0,2 | | | 0,3 |
| Kreatinin | | | 0,01 | | 0,02 | |
| Creatin | | | 0,1 | | 0,2 | |
| PEG-40 Hydriertes Ricinusöl | | 0,5 | 0,3 | | | 0.5 |
| Trinatrium EDTA | 0,3 | 0,2 | 0,2 | 0,2 | 0,2 | 0,5 |
| Konservierungsmittel | q.s. | q.s. | q.s. | q.s. | q.s. | q.s. |
| Natriumhydroxid | q.s. | q.s. | q.s. | q.s. | q.s. | q.s. |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

### Beispiel 13: kosmetische Schäume - Zahlenangaben in Gew.-%

| **Emulsion** | **A** | **B** | **C** |
|---|---|---|---|
| Stearinsäure | 2 | 2 | |
| Palmitinsäure | | | 1,5 |
| Cetylalkohol | 2,5 | 2 | |
| Stearylalkohol | | | 3 |
| PEG-100 Stearat | | | 3,5 |
| PEG-40 Stearat | | 2 | |
| PEG-20 Stearat | 3 | | |
| Sorbitanstearat | | 0,8 | |
| C₁₂₋₁₅ Alkyl Benzoat | 5 | | |
| C₁₂₋₁₃ Alkyl Tartrat | | | 7 |
| Butylenglycol Dicaprylat/Dicaprat | | 6 | |
| Dicaprylyl Ether | | | 2 |
| Cyclomethicon | | 2 | 3 |
| Butylenglycol | 1 | | |
| Isohexadecan | 2 | | |
| Methylpropandiol | | | |
| Propylenglykol | | | 5 |
| Glycerin | 5 | 7 | |
| UVASorb® K2A | | | 2 |
| Uvinul® A Plus | 2 | 3 | |
| 2,4-dimethoxyzimtsäure-6-O-ascorbat | 0,5 | 1 | 1,5 |
| 4-methoxyzimtsäure-6-O-ascorbat | 1,5 | 1 | 0,5 |
| Parsol SLX^{®} | | 3 | |
| Homosalat | | 5 | |
| Phenylbenzimidazol Sulfonsäure | | 2 | 2 |
| Benzophenon-3 | 2 | | |
| Octylsalicylat | | 5 | |
| Octocrylen | 2 | | |
| Bis-Ethylhexyloxyphenol Methoxyphenyltriazin | | 3 | |
| 2,2'-Methylen-bis-(6-(2H-benzotriazol-2-yl)-4-(1,1,3,3-tetramethylbutyl)-phenol) | | | 8 |
| 2,4,6-Tris-(biphenyl)-1,3 5-triazin | 5 | | 4 |
| C8-C16 Alkylpolyglycoside | 1 | | |
| Vitamin E Acetat | 0,6 | 0,5 | 0,2 |
| Kreatin/Kreatinin | | | 0,5 |
| BHT | | | 0,1 |
| Na₂H₂EDTA | 0,50 | | |
| Parfum, Konservierungsmitte | q.s. | q.s. | q.s. |
| Farbstoffe, usw. | q.s. | q.s. | q.s. |
| Natriumhydroxid | q.s. | | q.s. |
| Kaliumhydroxid | | q.s. | |
| Wasser | ad 100,0 | ad 100,0 | ad 100,0 |

### Beispiel 14: kosmetische Schäume - Zahlenangaben in Gew.-%

| **Emulsion** | **D** | **E** | **F** | **G** |
|---|---|---|---|---|
| Stearinsäure | 2 | | | |
| Palmitinsäure | | | 3 | 3 |
| Cetylalkohol | 2 | 2 | | |
| Cetylstearylalkohol | | | 2 | 2 |
| Stearylalkohol | | | | |
| PEG-100 Stearat | | 4 | | |
| PEG-40 Stearat | 2 | | | |
| PEG-20 Stearat | | | 3 | 3 |
| Sorbitanstearat | 0,8 | | | |
| Tridecyl Trimellitate | | 5 | | |
| C₁₂₋₁₅ Alkyl Benzoat | | | 3 | 3 |
| Butylenglycol Dicaprylat/Dicaprat | 8 | | | |
| Octyldodecanol | | 2 | | |
| Cocoglyceride | | | | 2 |
| Dicaprylyl Ether | | | 2 | 2 |
| Cyclomethicon | | | | |
| Dimethicon | 1 | | 2 | 2 |
| Isohexadecan | | 3 | | |
| Methylpropandiol | | 4 | | |
| Propylenglykol | | | | |
| Glycerin | 5 | | 6 | 6 |
| NeoHeliopan^{®} AP | | 2 | | |
| Phenylbenzimidazol Sulfonsäure | 1 | | | 1 |
| 2,4,6-trimethoxyzimtsäure-6-O-ascorbat | 0,75 | 1,5 | 3,0 | 6,0 |
| 4-methoxyzimtsäure-6-O-ascorbat | 6 | 3 | 1,5 | 0,75 |
| Ethylhexylmethoxycinnamat | 5 | | 4 | 4 |
| Ethylhexyltriazon | | 2 | | 1 |
| Eusolex T-AVO^{®} | 2 | | | |
| Diethylhexylbutamidotriazon | 1 | | | |
| Butyl Methoxydibenzoylmethan | 2,5 | | 2 | 2 |
| Bis-Ethylhexyloxyphenol Methoxy-phenyltriazin | 2 | | | |
| Vitamin E Acetat | 0,2 | | 0,3 | 0,3 |
| Na₂H₂EDTA | | | | |
| Parfum, Konservierungsmittel | | | | |
| Farbstoffe, usw. | | | | |
| Natriumhydroxid | | q-s. | q.s. | |
| Triethanolamin | q.s. | | | q.s. |
| Wasser | ad 100,0 | ad 100,0 | ad 100,0 | ad 100,0 |

### Beispiel 15: Sonnenschutzlotionen (O/W) - Zahlenangaben in Gew.-%

| **Emulsion** | **A** | **B** | **C** | **D** | **E** | **F** |
|---|---|---|---|---|---|---|
| A | | | | | | |
| Octocrylene | 1,5 | 2 | | | | |
| Polycrylene | | | 3 | 3 | | |
| Bis-ethylhexylhydroxydimethoxybenzylmal onat (RonaCare AP) | 1,5 | | 1 | 1 | 1,5 | 1 |
| Bis-ethylhexylhydroxydimethoxybenzyliden emalonat (Oxynex ST) | | 1 | | | 1,5 | |
| 1-(4-tert-butylphenyl)-3-(4-methoxyphenyl)propan-1,3-dion (Eusolex 9020) | | | | | | 2 |
| 2,4-dimethoxyzimtsäure-6-O-ascorbat | 2 | | | 1 | 0,5 | 1,5 |
| 4-methoxyzimtsäure-6-O-ascorbat | | 2 | 1 | | 1,5 | 0,5 |
| C12-15 Alkyl benzoate | 2 | | | 2 | 1 | 2 |
| Dioctyl adipate | 1 | 1 | | 3 | | |
| Dimethicone | 1 | 1 | | | 1 | |
| Isopropylalcohol | 3 | 3 | 3 | 3 | 3 | 3 |
| Cocoglycerides | 4 | 5 | 6 | 3,5 | 8 | |
| (e.g. Myritol 331) | | | | | | |
| Capric/caprylic Triglyceride | 4 | 5 | 6 | 3,5 | 2 | 10 |
| (e.g. Mygliol 812) | | | | | | |
| Glyceryl stearate, cetyl alcohol, PEG-75 stearate, ceteth-20, steareth-20 | 3,3 | 3,3 | 3,3 | 3,3 | 3,3 | 3,3 |
| PPG-1-PEG-9 lauryl glycol ether | 0,5 | 0,5 | 0,5 | 0,5 | 0,5 | 0,5 |
| Diisostearoyl trimethylolpropane siloxy silicate | 1,5 | 1,5 | 1,5 | 1,5 | 1,5 | 1,5 |
| B | | | | | | |
| Ectoin | 0,1 | 0,1 | 0,1 | 0,1 | 0,1 | 0,1 |
| Allantoin | 0,2 | 0,2 | 0,2 | 0,2 | 0,2 | 0,2 |
| Dimethicon copolyol phosphate | 2,5 | 2,5 | 2,5 | 2,5 | 2,5 | 2,5 |
| Butylene glycol | 2,5 | 2,5 | 2,5 | 2,5 | 2,5 | 2,5 |
| Wasser | Ad 100 | Ad 100 | Ad 100 | Ad 100 | Ad 100 | Ad 100 |
| C | | | | | | |
| PPG-1 trideceth-6, polyquaternium-37, propylene glycol dicaprylate/dicaprate | 0,5 | | 1 | | | |
| D | | | | | | |
| Propylene glycol, DMMDM hydantoin, ehtylparaben | 0,7 | 5 | 7 | 10 | 3 | 3 |
| Parfüm | 0,3 | | 1 | | 1 | |

### Herstellung:

Zunächst werden die erfindungsgemäßen Substanzen in Isopropanol (alternativ Ethanol) vorgelöst bzw. vordispergiert. Ggbf. werden weitere oder alternative Alkohole und Glycole eingesetzt, wie z.B. Ethanol, Glycerin oder Decyl alkohol. Diese Vorlösung wird zu den weiteren Komponenten der Phase A gegeben. Anschließend wird Phase A auf 50-60 °C erhitzt. Nun Phase B auf 60 °C erhitzen, dann Phase C unter Rühren eindispergieren. Phase A unter kräftigem Rühren in die Phase B/C einrühren. Unter Rühren abkühlen und bei 40 °C Phase D zugeben. Homogenisieren und unter Rürhen auf 25 °C abkühlen. Anmerkung: Beschriebene Vorlösung kann alternativ auch unter Rühren bei Raumtemperatur der fertigen Emulsion zugesetzt werden. Da erfindungsgemäße Stoffe oxidationsempfindliche Gruppen aufweisen, ist es empfehlenswert, unter möglichst inerten Bedingungen zu formulieren bzw. zu produzieren.

### Beispiel 16: Haarfarbe aus verschiedenen Komponenten:

### Konponente A:

Tocopherol, Linalool, Geraniol, Disodium EDTA, Parfum, Toluene-2,5-diamine sulfate, ascorbic acid, alcohol denat., Sodium sulfite, Sodium hydroxide, Sodium cocoyl isethionate, Bis-ethylhexyl Hydroxydimethoxy Benzylmalonate, 2-Methylresorcinol, 6-Amino-m-cresol, 4-Amino-2-hydroxy-toluol, 4-Amino-m-cresol, Sodium lauryl sulfate, Ammonia, Lanolin alcohol, Glycol distearate, Sodium laureth sulfate, Glyceryl stearate, Ceteary alcohol, Aqua.

### Komponente B:

Aqua, hydrogen peroxide, cetearyl alcohol, PPG-38-buteth-37, petrolatum, laureth-2, sodium cetearyl sulfate, salicylic acid, disodium phosphate, phosphoric acid, etidronic acid.

### Komponente C:

Ethanolische Lösung von 2,4-Dimethoxyzimtsäure-6-O-ascorbat (2 Gew.-%) zudem enthaltend 4-methoxyzimtsäure-6-O-ascorbat (2 Gew.-%) sowie Bis-ethylhexyl Hydroxydimethoxy Benzylmalonate (1 Gew.-%).

### Anwendung:

Zur Haarfärbung wird bevorzugt in folgender Reihenfolge vorgegangen: Zunächst wird das Haar mit Komponente C vorbehandelt, Anschließend werden die Komponenten B und C gemischt und auf das Haar appliziert. Eine weitere Applikationsvariante sieht vor, 2,4-dimethoxyzimtsäure-6-O-ascorbat und 4-methoxyzimtsäure-6-O-ascorbat direkt in Komponente A zu integrieren.

## Patentansprüche

1. Verbindungen der Formel I 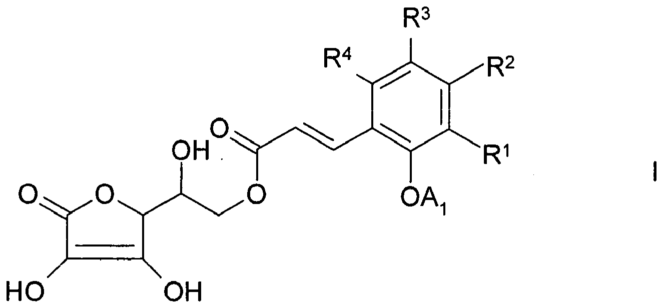 wobei
A₁ steht für H oder eine geradkettige oder verzweigte Alkylgruppe mit 1 bis 20 C-Atomen,
R¹ bis R⁴ stehen jeweils unabhängig voneinander für H, geradkettige oder verzweigte Alkoxygruppen mit 1 bis 20 C-Atomen, Hydroxy, fluorierte geradkettige oder verzweigte Alkoxygruppen mit 1 bis 20 C-Atomen oder Alkylcarbonyloxy und
Alkylcarbonyloxy steht für Alkyl-C(=O)-O, wobei Alkyl eine geradkettige oder verzweigte Alkylgruppe mit 1 bis 10 C-Atomen bedeutet.

2. Verbindungen nach Anspruch 1, **dadurch gekennzeichnet, dass** A₁ eine geradkettige oder verzweigte Alkylgruppe mit 1 bis 4 C-Atomen bedeutet.

3. Verbindungen nach Anspruch 1 oder 2, **dadurch gekennzeichnet, dass** der Substituent R² Hydroxy oder eine geradkettige oder verzweigte Alkoxygruppe mit 1 bis 4 C-Atomen bedeutet.

4. Verbindungen nach einem oder mehreren der Ansprüche 1 bis 3, **dadurch gekennzeichnet, dass** mindestens ein weiterer Substituent ausgewählt aus R¹, R³ oder R⁴ für Hydroxy oder eine geradkettige oder verzweigte Alkylgruppe mit 1 bis 20 C-Atomen steht.

5. Verfahren zur Herstellung von Verbindungen der Formel I nach einem oder mehreren der Ansprüche 1 bis 4, **dadurch gekennzeichnet, dass** Ascorbinsäure mit einer Verbindung der Formel II, 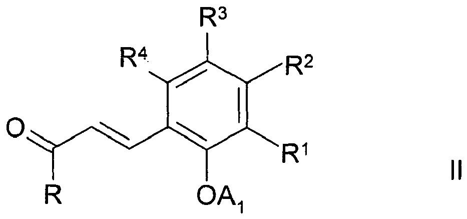 verestert wird,
wobei R OH, Halogen oder ein Aktivester bedeutet, Halogen Cl, Br oder I bedeutet und die Substituenten A₁, R¹ bis R⁴ eine in einem der Ansprüche 1 bis 3 angegebene Bedeutung haben.

6. Zubereitung enthaltend mindestens eine Verbindung nach einem oder mehreren der Ansprüche 1 bis 4.

7. Zubereitung nach Anspruch 6, **dadurch gekennzeichnet, dass** die mindestens eine Verbindung der Formel I in Mengen von 0,05 bis 10 Gew.-% enthalten ist.

8. Zubereitung nach Anspruch 6 oder 7, **dadurch gekennzeichnet, dass** mindestens ein weiterer organischer UV-Filter enthalten ist.

9. Zubereitung nach einem oder mehreren der Ansprüche 6 bis 8, **dadurch gekennzeichnet, dass** mindestens ein anorganischer UV-Filter enthalten ist.

10. Zubereitung nach einem oder mehreren der Ansprüche 6 bis 9, **dadurch gekennzeichnet, dass** mindestens ein weiteres Ascorbinsäurederivat, bevorzugt aus der Gruppe Ascorbinsäure, Magnesiumascorbylphosphat oder Ascorbylpalmitat, enthalten ist.

11. Zubereitung nach einem oder mehreren der Ansprüche 6 bis 10, **dadurch gekennzeichnet, dass** mindestens ein weiterer kosmetischer Wirkstoff enthalten ist, ausgewählt aus Antioxidantien, Anti-aging-Wirkstoffen, Anti-Cellulite-Wirkstoffen, Selbstbräunungssubstanzen, hautaufhellenden Wirkstoffen oder Vitaminen.

12. Verfahren zur Herstellung einer Zubereitung nach einem oder mehreren der Ansprüche 6 bis 10, **dadurch gekennzeichnet, dass** mindestens eine Verbindung nach einem oder mehreren der Ansprüche 1 bis 4 mit einem Träger und gegebenenfalls mit weiteren Aktiv- oder Hilfsstoffen vermischt wird.

13. Nicht-therapeutische Verwendung von Verbindungen der Formel I nach einem oder mehreren der Ansprüche 1 bis 4 als haut- und/oder haarbindender UV-Filter.

14. Verwendung von Verbindungen der Formel I nach einem oder mehreren der Ansprüche 1 bis 4 zur Verhinderung des oxidativen Abbaus von Farbstoffen, Parfümkomponenten oder Vitaminen.

## Claims

1. Compounds of the formula I 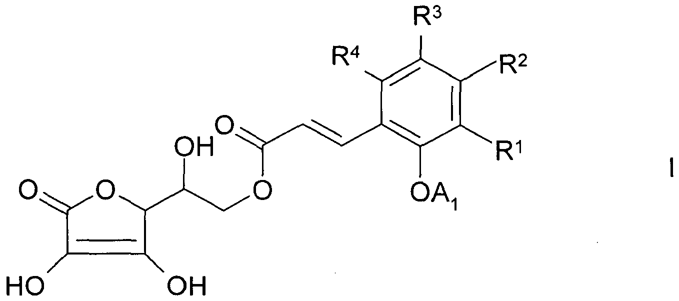 where
A₁ stands for H or a straight-chain or branched alkyl group having 1 to 20 C atoms,
R¹ to R⁴ each stand, independently of one another, for H, straight-chain or branched alkoxy groups having 1 to 20 C atoms, hydroxyl, fluorinated straight-chain or branched alkoxy groups having 1 to 20 C atoms or alkylcarbonyloxy, and
alkylcarbonyloxy stands for alkyl-C(=O)-O, where alkyl denotes a straight-chain or branched alkyl group having 1 to 10 C atoms.

2. Compounds according to Claim 1, **characterised in that** A₁ denotes a straight-chain or branched alkyl group having 1 to 4 C atoms.

3. Compounds according to Claim 1 or 2, **characterised in that** the substituent R² denotes hydroxyl or a straight-chain or branched alkoxy group having 1 to 4 C atoms.

4. Compounds according to one or more of Claims 1 to 3, **characterised in that** at least one further substituent selected from R¹, R³ or R⁴ stands for hydroxyl or a straight-chain or branched alkyl group having 1 to 20 C atoms.

5. Process for the preparation of compounds of the formula I according to one or more of Claims 1 to 4, **characterised in that** ascorbic acid is esterified using a compound of the formula II, 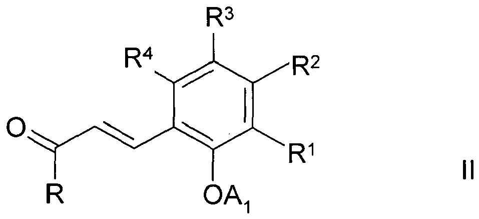 where R denotes OH, halogen or an active ester, halogen denotes Cl, Br or I, and the substituents A₁, R¹ to R⁴ have a meaning indicated in one of Claims 1 to 3.

6. Preparation comprising at least one compound according to one or more of Claims 1 to 4.

7. Preparation according to Claim 6, **characterised in that** the at least one compound of the formula I is present in amounts of 0.05 to 10% by weight.

8. Preparation according to Claim 6 or 7, **characterised in that** at least one further organic UV filter is present.

9. Preparation according to one or more of Claims 6 to 8, **characterised in that** at least one inorganic UV filter is present.

10. Preparation according to one or more of Claims 6 to 9, **characterised in that** at least one further ascorbic acid derivative, preferably from the group ascorbic acid, magnesium ascorbyl phosphate or ascorbyl palmitate, is present.

11. Preparation according to one or more of Claims 6 to 10, **characterised in that** at least one further cosmetic active compound is present, selected from antioxidants, anti-ageing active compounds, anti-cellulite active compounds, self-tanning substances, skin-lightening active compounds or vitamins.

12. Process for the preparation of a preparation according to one or more of Claims 6 to 10, **characterised in that** at least one compound according to one or more of Claims 1 to 4 is mixed with a carrier and optionally with further active compounds or assistants.

13. Non-therapeutic use of compounds of the formula I according to one or more of Claims 1 to 4 as UV filters which bond to the skin and/or hair.

14. Use of compounds of the formula I according to one or more of Claims 1 to 4 for preventing oxidative degradation of dyes, perfume components or vitamins.

## Revendications

1. Composés de formule I 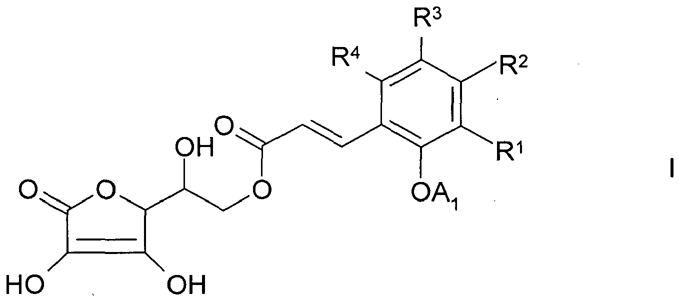 dans laquelle
A₁ représente H ou un groupement alkyle à chaîne linéaire ou ramifiée ayant de 1 à 20 atomes de C,
R¹ à R⁴ représentent chacun, indépendamment les uns des autres, H, des groupements alcoxy à chaîne linéaire ou ramifiée ayant de 1 à 20 atomes de C, hydroxyle, des groupements alcoxy fluorés à chaîne linéaire ou ramifiée ayant de 1 à 20 atomes de C ou alkylcarbonyloxy, et
alkylcarbonyloxy représente alkyl-C(=O)-O, où alkyle désigne un groupement alkyle à chaîne linéaire ou ramifiée ayant de 1 à 10 atomes de C.

2. Composés selon la revendication 1, **caractérisés en ce que** A₁ désigne un groupement alkyle à chaîne linéaire ou ramifiée ayant de 1 à 4 atomes de C.

3. Composés selon la revendication 1 ou 2, **caractérisés en ce que** le substituant R² désigne hydroxyle ou un groupement alcoxy à chaîne linéaire ou ramifiée ayant de 1 à 4 atomes de C.

4. Composés selon l'une ou plusieurs parmi les revendications 1 à 3, **caractérisés en ce qu'**au moins un autre substituant choisi parmi R¹, R³ ou R⁴ représente hydroxyle ou un groupement alkyle à chaîne linéaire ou ramifiée ayant de 1 à 20 atomes de C.

5. Procédé de préparation de composés de formule I selon l'une ou plusieurs parmi les revendications 1 à 4, **caractérisé en ce que** de l'acide ascorbique est estérifié en utilisant un composé de formule II, 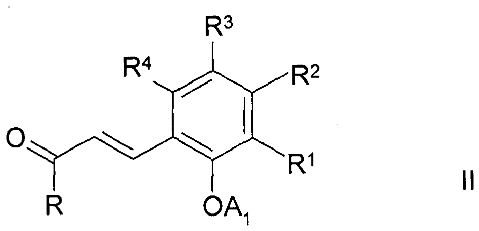 où R désigne OH, halogène ou un ester actif, halogène désigne Cl, Br ou I, et les substituants A₁, R¹ à R⁴ revêtent une signification indiquée selon l'une des revendications 1 à 3.

6. Préparation comprenant au moins un composé selon l'une ou plusieurs parmi les revendications 1 à 4.

7. Préparation selon la revendication 6, **caractérisée en ce que** le au moins un composé de formule I est présent selon des quantités allant de 0,05 à 10% en poids.

8. Préparation selon la revendication 6 ou 7, **caractérisée en ce qu'**au moins un autre filtre anti-UV organique est présent.

9. Préparation selon l'une ou plusieurs parmi les revendications 6 à 8, **caractérisée en ce qu'**au moins un filtre anti-UV inorganique est présent.

10. Préparation selon l'une ou plusieurs parmi les revendications 6 à 9, **caractérisée en ce qu'**au moins un autre dérivé d'acide ascorbique, issu préférablement du groupe constitué par l'acide ascorbique, le phosphate d'ascorbyle et de magnésium ou le palmitate d'ascorbyle, est présent.

11. Préparation selon l'une ou plusieurs parmi les revendications 6 à 10, **caractérisée en ce qu'**au moins un autre composé actif cosmétique est présent, choisi parmi les antioxydants, les composés actifs antivieillissement, les composés actifs anticellulite, les substances auto-bronzantes, les composés actifs d'éclaircissement de la peau ou les vitamines.

12. Procédé de préparation d'une préparation selon l'une ou plusieurs parmi les revendications 6 à 10, **caractérisé en ce qu'**au moins un composé selon l'une ou plusieurs parmi les revendications 1 à 4 est mélangé avec un véhicule et éventuellement avec d'autres composés actifs ou des auxiliaires.

13. Utilisation non thérapeutique de composés de formule I selon l'une ou plusieurs parmi les revendications 1 à 4, comme filtres anti-UV qui se fixent à la peau et/ou aux cheveux.

14. Utilisation de composés de formule I selon l'une ou plusieurs parmi les revendications 1 à 4, pour la prévention de la dégradation oxydative de colorants, de composants de parfum ou de vitamines.
